# EUROPÄISCHE PATENTSCHRIFT

(11) **EP 2 110 132 B1**
(45) Veröffentlichungstag und Bekanntmachung des Hinweises auf die Patenterteilung: **22.01.2014**
(21) Anmeldenummer: 08007676.3
(22) Anmeldetag: 20.04.2008
(51) Int. Cl.: A61K 33/00, A61P 29/00, A61P 37/08, A61P 11/00, A61P 9/00

(54) **Verwendung von Deuteriumoxid als Elastase-Inhibitor**
Use of deuterium oxide as elastase inhibitor
Utilisation d'oxyde de deutérium en tant qu'inhibiteur d'élastase

(43) Veröffentlichungstag der Anmeldung: 21.10.2009
(60) Teilanmeldung: 12166154.0
(73) Patentinhaber: D2 Bioscience Group Ltd, Hamilton HMLX (BM)
(72) Erfinder: Bayerl, Thomas, Prof. Dr., London SW1X0DE (GB)
(74) Vertreter: Ettmayr, Andreas

(56) Entgegenhaltungen:
- EP-A- 0 893 123
- WO-A-99/62510
- WO-A-2007/129962
- DE-A1- 4 427 690
- US-A- 5 223 269
- KUSHNER DJ ET AL: "Pharmacological uses and perspectives of heavy water and deuterated compounds" CANADIAN JOURNAL OF PHYSIOLOGY AND PHARMACOLOGY, OTTAWA, ONT, CA, Bd. 77, Nr. 2, 1. Februar 1999 (1999-02-01), Seiten 79-88, XP009086918
- WHITE R R ET AL: "Effect of Colchicine, vinblastine, D2O and cytochalasin B on elastase secretion, protein synthesis and fine structure of mouse alveolar macrophages." JOURNAL OF THE RETICULOENDOTHELIAL SOCIETY APR 1981, Bd. 29, Nr. 4, April 1981 (1981-04), Seiten 295-304, XP009106069 ISSN: 0033-6890

## Beschreibung

Die vorliegende Erfindung betrifft die Verwendung von Deuteriumoxid (D20) als Elastase-Inhibitor und insbesondere als Inhibitor der humanen neutrophilen Elastase (HNE). Weiterhin betrifft die Erfindung die Verwendung von Deuteriumoxid zur Prophylaxe und/oder Therapie von HNE-assoziierten Erkrankungen.

Elastasen gehören zu der Familie der Serinproteasen und spalten Amide, somit auch Amidbindungen von Peptiden und Proteinen, und Ester unter Aufnahme von H2O. Humane Elastasen werden von sechs verschiedenen Genen kodiert und umfassen die humane Leukozyten-Elastase (HLE), auch bekannt als humane neutrophile Elastase (HNE), (EC 3.4.21.37). Die humane neutrophile Elastase (HLE) ist eine glykolysierte, basische Serinprotease mit 218 Aminosäuren mit einem Molekulargewicht von ungefähr 33 kDa. HNE kommt in den azurophilen Granula der humanen polymorphkernigen Leukozyten (engl. polymorphnuclear leucocyte, PMN) vor. Die intrazelluläre physiologische Funktion der HNE besteht in der Degradierung von organischen Fremdpartikeln, die über die Phagozytose aufgenommen werden. Nach Aktivierung der polymorphkernigen Leukozyten wird die Elastase HNE aus diesen in den extrazellulären Raum freigesetzt (freie Elastase), wobei ein Anteil an die neutrophile Plasmamembran der PMN gebunden verbleibt (membranständige Elastase). Die aktive extrazelluläre HNE ist in der Lage nahezu alle extrazellulären Matrixproteine zu hydrolysieren, wie beispielsweise Elastin, Collagen, Laminin, Zytokine und Fibronektin.

Aufgrund ihrer unkontrollierten proteolytischen Aktivität spielt HNE jedoch eine destruktive Rolle bei einer Vielzahl pathologischer Prozesse. Hierzu gehören insbesondere Entzündungskrankheiten, z.B. Entzündungskrankheiten der Haut, wie neutrophile Dermatosen, wie palmoplantar pustolosis, subcorneal pustolosis (Sneddon-Wilkinson's Krankheit) (Glinski W. et al., Basement membrane zone as a target for human neutrophil elastase in psoriasis, Arch Dermatol Res. 1990;282(8):506-11; Meyer-Hoffert U. et al., Human leukocyte elastase induces keratinocyte proliferation by epidermal growth factor receptor activation, J Invest Dermatol. 2004 Aug;123(2):338-45: Wiedow O. et al., Lesional elastase activity in psoriasis. Diagnostic and prognostic significance, Arch Dermatol Res. 1995;287(7):632-5), autoimmune bullöse Dermatose, Pemphigoid, wie bullöses Pemphigoid, Pemphigoid vulgaris, Pemphigoid vegetans und Pemphigoid foliaceus (Oikarinen Al et al., Demonstration of collagenase and elastase activities in the blister fluids from bullous skin diseases. Comparison between dermatitis herpetiformis and bullous pemphigoid, J Invest Dermatol. 1983 Sep;81(3):261-6; sLiu Z. et al., A critical role for neutrophil elastase in experimental bullous pemphigoid, J Clin Invest. 2000 Jan;105(1):113-23; Shimanovich I. et al., Granulocyte-derived elastase and gelatinase B are required for dermal-epidermal separation induced by autoantibodies from patients with epidermolysis bullosa acquisita and bullous pemphigoid, J Pathol. 2004 Dec; 204(5):519-27), Aphthöse Erkrankungen der Mundschleimhaut, Entzündung der Nasennebenhöhlen, der Nasenschleimhaut, des Mittelohrs, der Augenbindehaut sowie Heuschnupfen, Asthma, cutane Vaskularitis, Lungenvaskularitis, Peritonitis und septischer Schock (Tsujimoto H. et al., Neutrophil elastase, MIP-2, and TLR-4 expression during human and experimental sepsis, Shock. 2005 Jan;23(1):39-44; Jochum M. et al., Proteolytic destruction of functional proteins by phagocytes in human peritonitis, Eur J Clin Invest. 1999 Mar;29(3):246-55) sowie Lungenerkrankungen, z.B. chronische obstruktive Lungenerkrankung (COPD), zystische Fibrose, chronische Bronchitis, Lungenfibrose, akutes Atemwegssyndrom, Lungenemphysem (Yoshioka, A. et ai, Excessive neutrophil elastase in bronchoalveolar lavage fluid in subclinical emphysema. Am. J. Respir. Crit. Care Med., Dec 1995; 152: 2127.) Hämorrhagie (Lungenschädigung) etc., Herz- und Kreislauferkrankungen, wie Herzinfarkt, Hirnischämie (cerebrale Ischämie) sowie andere Gewebsveränderungen nach einem Herzinfarkt, Herzinsuffizienz und akutes Koronarsyndrom (Durchblutungsstörungen der Herzkranzgefäße) (Lechleitner P. et al, Granulocyte elastase in acute myocardial infarction, Z Kardiol. 1993 Oct;82(10):641-7; Dinerman JL et al., Increased neutrophil elastase release in unstable angina pectoris and acute myocardial infarction, J Am Coll Cardiol. 1990 Jun;15(7):1559-63; Tiefenbacher CP et al., Inhibition of elastase improves myocardial function after repetitive ischaemia and myocardial infarction in the rat heart, Pflugers Arch. 1997 Mar;433(5):563-70; Bidouard JP et al., SSR69071, an elastase inhibitor, reduces myocardial infarct size following ischemia-reperfusion injury, Eur J Pharmacol. 2003 Feb 7;461(1):49-52; Ohta K. et al., Elafin-overexpressing mice have improved cardiac function after myocardial infarction, Am J Physiol Heart Circ Physiol. 2004 Jul;287(1):H286-92; Shimakura A. et al., Neutrophil elastase inhibition reduces cerebral ischemic damage in the middle cerebral artery occlusion; Brain Res. 2000 Mar 6;858(1):55-60.) und allergische Erkrankungen, wie Allergie gegen Hausstaub, Milben, Pflanzenpollen, allergisches Asthma etc. (Sehgal N. et al., Potential roles in rhinitis for protease and other enzymatic activities of allergens, Curr Allergy Asthma Rep. 2005 May;5(3):221-6; Gunawan H. et al., Protease Activity of Allergenic Pollen of Cedar, Cypress, Juniper, Birch, and Ragweed, Allergol Int. 2008 Mar 1;57(1):83-91; Runswick S. et al., Pollen proteolytic enzymes degrade tight junctions, Respirology. 2007 Nov;12(6):834-42.

Die Rolle von Serinproteasen insgesamt, wie Elastasen, in pathogenen Prozessen ist wahrscheinlich in einem Ungleichgewicht des Enzyms und seines natürlichen körpereigenen Inhibitor begründet. Im Fall von HNE erfolgt die Inhibierung des Enzyms durch das Inhibitorprotein alpha1-Antitrypsin, A1AT, inhibiert (Stockley, Neutrophils and protease-antiprotease imbalance, AmJ.Respir.Crit.Care Med 1999; 160, 49-52). A1AT wird normalerweise von der Leber ins Serum sekretiert und liegt in hohem Überschuß zu HNE vor und bindet irreversibel an das aktive Zentrum von HNE und Trypsin. Auf diese Weise wird das freie Enzym HNE deaktiviert. Sofern jedoch ein Mangel an A1AT vorliegt, führt dies zu einem Überschuß an aktiver HNE und einer unkontrollierten Aktivität des Enzyms. Zudem ist das membranständige Enzym HNE für A1AT nicht zugänglich.

In den letzten Jahren wurde zunehmend nach spezifischen Serinprotease-Inhibitoren gesucht. Es wurden zahlreiche Inhibitoren für Elastasen, einschließlich der HNE, meist auf Basis kleiner Moleküle mit MW > 1 kD, vorgeschlagen und untersucht (Edwards, P. D.; Bernstein, P. R. Synthetic inhibitors of elastase. Med. Res. Rev. 1994, 14, 127-94; J. Potemp et al., The Serpin Superfamily of Proteinase Inhibitors: Structure, Function, and Regulation, J. Biol. Chem Vol. 269 No. 23, Issue of June 10, pp. 15957-15960 1994).

Solche Elastase-Inhibitoren können allgemein in die Gruppen irreversible und reversible Inhibitoren unterteilt werden. Während irreversible Elastase-Inhibitoren, wie HNE-Inhibitoren (u.a. Alkylfluorophosphate, Chloromethylketone, Sulfonylfluoride), meist eine kovalente Bindung mit der Substratbindungsstelle der Elastasen bzw. HNE eingehen, sind reversible Elastase- bzw. HNE-Inhibitoren meist durch Wasserstoffbrücken, ionische Bindungen oder van der Waals Wechselwirkungen zwischen Inhibitor und der Elastase bzw. HNE gekennzeichnet und weisen häufig eine elektrophile funktionelle Gruppe am C-Terminus des P1 Residuums auf, welche ihnen eine höhere Bindungsaffinität ermöglicht (u.a. Trifluoromethylketone, Borsäureester, Aldehyde).

Für die Entwicklung von Elastase-Inhibitoren und insbesondere HNE-Inhibitoren zur Therapie von Krankheiten werden reversible Inhibitoren wegen ihrer potentiell geringeren toxischen Nebenwirkungen bevorzugt. Trotzdem weisen auch diese reversiblen Inhibitoren noch beträchtliche Nachteile bei der Anwendung am tierischen Organismus auf. Aldehyde unterliegen beispielsweise der Gefahr einer raschen Oxidation zu Carboxylsäuren sowie der Racemisierung in Anwesenheit von Säuren oder Basen, wenn ein chirales Zentrum am Alpha-Kohlenstoff des P1 Residuums vorhanden ist. Weiterhin ist ihre geringe Bioverfügbarkeit, insbesondere ihre geringe orale Bioverfügbarkeit, aufgrund elektrophiler funktioneller Gruppen ein den Einsatz am tierischen Organismus, wie am Säugetier, limitierender Faktor.

Zahlreiche bekannte HNE-Inhibitoren sind peptidbasiert und bestehen aus nicht mehr als 3-5 Aminosäureresten bzw. ihrem Equivalent (S. Sinha et al., Conversion of the Alzheimer's @-Amyloid Precursor Protein (APP) Kunitz Domain into a Potent Human Neutrophil Elastase Inhibitor, J. Biol. Chem. Vol. 266, NO. 31, Issue of November 5, 21011-21013,1991) Wegen dieser geringen Größe können sie nur mit kleinen Bereichen des HNE-Enzyms in Wechselwirkung treten, was ihre Spezifität stark einschränkt. Beim Einsatz am tierischen Organismus, wie am Säugetier, lassen derartige Moleküle starke Nebenwirkungen erwarten. Auch verschiedene Sulfonamide, für welche eine starke inhibitorische Wirkung auf Serinproteasen insgesamt nachgewiesen wurde (Sommerhoff, C. P.; Krell, R. D.; Williams, J. L.; Gomes, B. C.; Strimpler, A. M.; Nadel, J. A. Inhibition of human neutrophil elastase by ICI 200,355. Eur. J. Pharmacol. 1991, 193, 153-8; M. Takayama et al., Effects of neutrophil elastase inhibitor (ONO-5046) on lung injury after intestinal ischemia-reperfusion , J Appl Physiol 91: 1800 - 1807, 2001), haben ein Spezifitätsproblem und weisen damit Nebenwirkungen beim Einsatz am tierischen Organismus, wie am Säugetier, auf. Für größere, ebenfalls meist peptidbasierte Moleküle mit inhibitorischer Wirkung für HNE kommen zwei weitere Probleme hinzu. Zum einen unterliegen sie der Gefahr einer nicht-spezifischen Proteolyse durch andere Proteasen (z.B. durch Cystein- oder Metalloprotesasen) und die Produkte dieser Reaktion können Nebenwirkungen verursachen (S. Attucci et al. , EPI-hNE4, a Proteolysis-Resistant Inhibitor of Human Neutrophil Elastase and Potential Anti-Inflammatory Drug for Treating Cystic Fibrosis, JPET 318:803-809, 2006). Außerdem sind diese höhermolekularen HNE-Inhibitoren wegen ihrer Molekülgröße nur beschränkt einsetzbar, da zu einer spezifischen Inhibierung der HNE eine flexible Konformation der an die katalytische Triade des Enzyms bindenden funktionellen Gruppe des Inhibitors/Substrats notwendig ist.

Nachteile der im Stand der Technik bekannten Elastase-Inhibitoren und insbesondere HNE-Inhibitoren sind demnach unter anderem eine unspezifische Wirkung, unerwünschte Nebenwirkungen, Racemierung, Oxidierung im stark oxidierenden Millieu, geringe Bioverfügbarkeit und die Gefahr der Proteolyse durch andere Proteasen, wodurch sie in ihrer inhibitorischen Aktivität gehemmt oder inaktivert werden.

DE4427690A1 beschreibt eine pharmazeutische Zusammensetzung, die Deuterium oder deuterierte Substanzen oder Substanzen, die Deuterium anreichern bzw. freisetzen, enthält zur Verwendung als Zytostatikum oder Tumortherapeutiku, für die Bekämpfung von Tumoren und Metastasen.

WO1999/062510A2 beschreibt Verfahren und Zusammensetzungen zur Behandlung von Entzündungserkrankungen mittels anti-mikrotubulärer Substanzen.

W02007/129962A1 beschreibt 2-Pyridonderivate für die Behandlung von Erkrankungen, bei denen eine Inhibierung der neutrophilen Elastaseaktivität vorteilhaft ist.

White et al. , Effect of Colchicine, vinblastine, D20 and cytochalasin B on elastase secretion, protein synthesis and fine structure of mouse alveolar macrophage", Journal of the Reticuloendothelial Society Apr 1981, Bd. 29, Nr. 4, 1981-04, S. 295-304 beschreibt die Wirkung von Cholchin, Vinblastin, D20 und Cytochalasin B auf die Elastasesekretion, Proteinsynthese und Feinstruktur von Alveolar-Makrophagen der Maus.

Aufgabe der vorliegenden Erfindung ist es daher, verbesserte Inhibitoren für Elastasen, und insbesondere verbesserte Inhibitoren für die humane neutrophile Elastase (HNE), bereitzustellen.

Diese Aufgabe wird mit der vorliegenden Erfindung gelöst. Die vorliegende Erfindung basiert auf der Erkenntnis, dass Deuteriumoxid (D20) ein effektiver Inhibitor für Elastasen, und insbesondere für die humane neutrophile Elastase, ist. Deuteriumoxid (D20), häufig auch als schweres Wasser bezeichnet, ist eine dem natürlichen Wasser (H2O) äußerst ähnliche Substanz. D20 und H2O unterscheiden sich physikalisch durch die Substitution der Wasserstoffatome durch Deuteriumatome. D20 weist eine ca. 10% höhere Dichte und eine ca. 25% höhere Viskosität als H2O auf. Darüber hinaus sind die Schmelz- und Siedetemperaturen von D20 höher als für H2O. Ein detaillierter Vergleich der Eigenschaften wird im Handbook of Chemistry and Physics, Sektion 6, gegeben (Handbook of Chemsitry and Physics, David R. Lide, Editor, 79. Auflage, 1998 CRC Press, Boca Raton, USA) dargestellt.

Während die physikalischen Unterschiede zwischen H2O und D20 eher gering sind, bestehen bedeutsame physiologische Unterschiede (siehe u.a. Kushner DJ et al., Pharmacological uses and perspectives of heavy water and denatured compounds., Can J Physiol Pharmacol. 1999 Feb;77(2):79-88.). So wurde beispielsweise festgestellt, dass, obwohl viele Algen und Bakterien in 100% D20 völlig normal über lange Zeiten existieren können, und für Protozoen dies bei bis zu 70% D20 möglich ist, dies nicht für tierische Zellen gilt. Hier werden bei D20-Konzentrationen im Organismus von mehr als 20-25% verschiedene enzymatisch gesteuerte Reaktionen zunehmend verändert oder inhibiert. Eine Ursache hierfür wird in der veränderten Bindungsstärke der WasserstoffBrückenbindungen, wenn das Wasserstoffatom durch ein Deuteriumatom substituiert ist, gesehen. Sowohl in wässrigen Lösungen von H2O und D2O, als auch bei der Bindung von Wasser an organische Moleküle, tritt diese, meist erhöhte Bindungsstärke auf, wobei bei organischen Molekülen der Effekt noch stärker ausgeprägt zu sein scheint (Cuma M, Scheiner S, Influenee of Isotopic Substitution on Strength of Hydrogen Bonds of Common Organic Groups, Journal of Physical Organic Chemistry, 1997 Band 10, 383-395).

Bislang galt im Stand der Technik ein therapeutischer Nutzen der Verabreichung von D20 an einen tierischen Organismus, insbesondere an ein Säugetiers, als wenig effektiv, da die für eine solche Wirkung erforderlichen D20-Konzentrationen über 25% liegen müssten und damit starke Nebenwirkungen erwartet wurden (Kushner DJ et al., Pharmacological uses and perspectives of heavy water and denatured compounds., Can J Physiol Pharmacol. 1999 Feb;77(2):79-88.). Darüber hinaus kommen alle im Stand der Technik publizierten Studien zur D20-Wirkung an Zellen, Organen und Organismen zu der Schlussfolgerung, dass die Wirkung von D20 nur für die Zeitdauer der D20-Verabreichung anhält, wodurch sehr lange Zyklen der D20-Verabreichung notwendig wären.

Bisherige Studien aus dem Stand der Technik beschäftigen sich vordringlich mit der antitumoralen und anti-neoplastischen Wirkung von D20.

Für den Fall des Tumorwachstums im Dickdarm und in den Plattenepithelzellen im Mund- und Rachenraum und wurde die Antitumor-Wirkung von D20 bereits in den 80-iger Jahren experimentell an Balb/c/-nu/nu Mäusen nachgewiesen (Altermatt HJ et al., Heavy water delays growth of human carcinoma in nude mice; Cancer. 1988 Aug 1;62(3):462-6.) Auch in anderen Geweben konnte eine Antitumorwirkung nachgewiesen werden. So unterstützen kürzlich publizierte Zellkultur-Studien (Hartmann, J. et al., Effects of heavy water (D20) on human pancreatic tumor cells, Anticancer Res. 2005 Sep-Oct;25(5):3407-11) mit 3 Tumorzellinien aus der Bauchspeicheldrüse ebenfalls eine Antitumor-Wirkung. Andere Untersuchungen fanden eindeutige Resultate für die D20-vermittelte Antitumor-Wirkung in Geweben von neoplastischen Hirnzellen (Uemura, T. et al, Experimental validation of deuterium oxide-mediated antitumoral activity as it relates to apoptosis in murine malignant astrocytoma cells, Neurosurg. 2002 May;96(5):900-8.) und legen nahe, dass D20 eine Apoptose in malignen Astrozytomzellen induzieren kann. Auch in dieser Arbeit schlussfolgerten die Autoren, dass D20 zytotoxisch auf Tumorgewebe wirkt und dadurch ein Zytostatikum darstellt.

Publizierte Studien, bei denen ein etabliertes Zytostatikum zusammen mit D20 anstelle von H2O zur Behandlung von murinen, xenotransplantierten Tumoren verabreicht wurde (Altermatt, HJ, Heavy water (D2O) inhibits growth of human xenotransplanted oropharyngeal cancers, An animal experiment study in nude mice, Laryngol Rhinol Otol (Stuttg). 1987 Apr;66(4):191-4.), bestätigen eine zusätzliche anti-neoplastische Wirkung von D20.

Nirgendwo im Stand der Technik ist bislang jedoch D20 als ein effektiver Inhibitor enzymatischer Aktivitäten, wie als Elastase-Inhibitor und vor allem als Inhibitor der humanen neutrophilen Elastase (HNE), beschrieben worden.

Der erfindungsgemäße Wirkmechanismus von D20 als Elastase-Inhibitor, insbesondere HNE-Inhibitor, basiert generell auf Veränderungen in der Wasserstoffbrücken-Bindungsenergie, wenn Wasserstoffatome solcher Bindungen durch Deuteriumatome ersetzt werden. Für Proteine und Enzyme ist dies in mehrfacher Hinsicht bedeutungsvoll. Wasserstoffbrückenbindungen (H-Brücken) stabilisieren deren Tertiär- und Quartärstruktur und beeinflussen damit die räumliche Anordnung der einzelnen Bereiche (Domänen) zueinander sowie deren (funktionelle) Veränderungen durch Faltungen.

Unter der Tertiärstruktur eines Proteins versteht man den der Sekundärstruktur (Aminosäurekette) übergeordneten räumlichen Aufbau von Proteinen, d.h. die vollständige dreidimensionale Struktur der Aminosäurekette, die für die biologische Funktion des Proteins unerlässlich ist. Bei der Faltung der Aminosäurekette zur dreidimensionalen Struktur des Proteins werden die hydrophoben Bereiche im Inneren des Proteins angeordnet, während die hydrophilen Bereiche nach außen zeigen und damit der wässrigen Umbebung des Proteins zugewandt sind. Die Sabilisierung der Tertiärstruktur eines Proteins findet über verschiedene Bindungen statt: den Disulfidbrücken, Ionenbindungen, Wasserstoffbrückenbindungen sowie hydrophoben Wechselwirkungen.

Die Quartärstruktur besteht aus der Zusammenlagerung mehrerer Proteinmoleküle zu einem funktionellen Komplex. Diese Zusammenlagerung erfolgt über nicht-kovalente Wechselwirkungen: Wasserstoffbrückenbindungen zwischen Peptidbindungen und Seitenketten, Ionenbindungen und von-der-Waals-Kräfte. Die Zusammenlagerung von Untereinheiten in hydrophoben Bereichen, demnach von Bereichen im Inneren des Proteins, erfolgt in erster Linie über den hydrophoben Effekt.

Durch Veränderungen der Wasserstoffbrücken-Bindungsenergien und damit der Stabilisierung des Proteins oder Enzyms durch H-Brücken werden Tertiär- und Quartärstruktur kann die Elastase, insbesondere die HNE, in eine Konformation gelangen, welche die Substratbindung sterisch verhindert oder energetisch ungünstig macht. Die Folge ist eine teilweise oder vollständige Inhibierung ihrer Aktivität.

Darüber hinaus sind H-Brücken in vielen Fällen bei der Bindung von Substraten und deren Modifikation durch Enzyme beteiligt. Eine Veränderung der Bindungsenergie kann hierbei vielfältige Auswirkungen haben, die als Konsequenz eine Veränderung des Reaktionsweges und/oder der Reaktionsgeschwindigkeit bewirken kann. Wie erwähnt, gehören Elastasen zu der Familie der Serinproteasen und zeichnen sich durch eine Spezifität für verzweigtkettige aliphatische Reste an P1 (Substrat) aus. Die katalytische Triade des Enzyms HNE besteht aus Ser 195, His 57 und Asp 102 Resten (Chymotrypsin-Nummerierung, siehe für Nomenklatur Greer J Comparative modeling methods: Application to the family of the mammalian serine proteases. Proteins Struct Funct Genet, 1990, 7:317-334) und einem Oxyanion -"Loch" im Substrataufnahme-Bereich. Das Substrat bindet unter Bildung eines Michaelis Komplexes, wobei die Carbonylgruppe der zu schneidenden Amidbindung an die Hydroxylgruppe des Ser 195 exponiert wird und einer basischen Katalyse durch die Imidazol Seitenkette des His 57 unterzogen wird. Das resultierende terahedrale Intermediat wird durch Wasserstoffbrücken stabilisiert, welche es an das NH-Gerüst von Ser 195 und Gly 193 binden. Anschließend erfolgt Wasseranlagerung an den Komplex, wodurch ein zweites tetrahedrales Intermediat gebildet wird und schießlich durch Säure-unterstützte Katalyse über His 57 abgebaut wird, unter Regenerierung von Ser 195 und dem N-terminalen Fragment des abgeschnittenen Substrats. In diesem katalytischen Prozess ist an drei essentiellen Schritten Wasserstoff involviert. Zunächst bei der Ausbildung einer H-Brücke zwischen Ser 195 und His 57, die erst bei Substratbindung erfolgt, und welche für die katalytische Wirksamkeit der Triade entscheidend ist (Perona J J and C S Craik, Structural basis of substrate specificity in the serine proteases, Protein Science (1995), 4:337-360). Weiterhin bei der Ausbildung der H-Brücken zur Intermediatstabilisierung und schließlich bei der katalytischen Wasseranlagerung an den Komplex. Da Deuterium-Brückenbindungen (D-Brücken) eine signifikant unterschiedliche Bindungsenergie im Vergleich zu H-Brücken haben (das zusätzliche Neutron im Deuterium schränkt einige hochfrequente Freiheitsgrade im Petahertz (10¹⁵ Hz) -Frequenzbereich ein, wodurch D-Brücken u.a. leicht verringerte Bindungsabstände haben), ist die katalytische Wirkung von His 57 sowie die Bindung via D-Brücken an Ser 195 und Gly 193 gestört. Der katalytische Umsatz wird verlangsamt oder vollständig inhibiert.

Ein weiterer Wirkmechanismus sind die durch Wasserstoff-Deuterium-Substitution in den zur Substratbindung notwendigen H-Brücken veränderten Bindungsabstände und Bindungsenergien. Hierdurch wird die spezifische Bindung von Substraten entweder erschwert bzw. unterbunden. Andererseits können bestimmte Substrate dadurch so fest an das Enzym gebunden werden, dass sie die katalytische Triade blockieren und dadurch die katalytische Aktivität des Enzyms inhibieren.

Ein dritter Wirkmechanismus ist die Substitution des Wasserstoffs in der H-Brücke zwischen His 57 und Asp 102 durch ein Deuteron. Die Veränderung in Bindungsenergie und Bindungsabstand führt effektiv zu einem Bruch dieser Bindung in der katalytischen Triade. Die Folgen eines derartigen Bindungsbruchs sind durch Molekulardynamiksimulationen (MD) eingehend untersucht worden und zeigten eindeutig, dass durch einen Bindungsbruch die katalytisch produktive Konformation der Triade verlorengeht (E Lau and T C Bruice , Consequences of Breaking the Asp-His Hydrogen Bond of the Catalytic Triad: Effects on the Structure and Dynamics of the Serine Esterase Cutinase , Biophysical Journal Vol 77, 1999 85-98).

Die hohe Spezifität von Deuteriumoxid als erfindungsgemäßen Elastase-Inhibitor, die vorstehend detailliert für die HNE gezeigt wird, wird zum einen durch die einzelnen aufgezählten Wirkmechanismen, zum anderen allerdings vor allem durch das verstärkende Zusammenwirken aller Mechanismen erzielt. Von den Trypsin-, Metallo- und Chymotrypsinproteasen unterscheidet sich HNE bezüglich der Substratspezifität dadurch, das für HNE diese nicht zusätzlich durch Bindungsmotive in Entfernung von S1 (d.h. S2-S4) determiniert ist. Damit fällt den H-Brücken und ihren Veränderungen durch die Deuterium-Substitution im Bereich von S1 der HNE einen wesentlich größere Bedeutung für die spezifische Substratbindung zu als bei den Trypsin-, Chymotrypsin- und Metalloproteasen. Auf diese Weise kann D20 spezifisch die Wirkung der HNE, inhibieren. Der für HNE beschriebene Wirkmechanismus läßt sich auf andere Elastasen übertragen.

Erfindungsgemäß wird durch die Verabreichung von D20 erreicht, dass die Bindungsenergien und Bindungsabstände der stabilisierenden Wasserstoffbrückenbindungen von Elastasen, vor allem der HNE, durch die Substitution von Wasserstoffatomen durch Deuteriumatome verändert wird. Durch diese höhere Bindungsstärke wird die Konformation des Enzyms verändert, was zu seiner teilweisen oder vollständigen Inaktivierung der katalytischen Aktivität führt. Mit anderen Worten, die enzymatische Aktivität der HNE wird ganz oder teilweise inhibiert. Die Spezifität für Elastasen, bzw. für HNE, wird durch die beschriebene charakteristische Triade (Ser 195/His 57/Asp 102) und die Konformationsänderung des Enzyms (durch Substitution von Wasserstoffatomen durch Deuteriumatome) erzielt.
Demnach ist D20 erfindungsgemäß ein effektiver und spezifischer Elastase-Inhibitor, vor allem ein Inhibitor der HNE, und somit in der Prophylaxe und/oder Therapie von Erkrankungen, die mit einer unspezifischen oder unkontrollierten Elastaseaktivität, vor allem der Aktivität der humanen neutrophilen Elastase assoziiert sind, wirkungsvoll.

Die vorliegende Erfindung betrifft Deuteriumoxid zur Verwendung für die Prophylaxe und/oder Therapie von Elastase-assozierten Lungenerkrankungen, ausgewählt aus der Gruppe, bestehend aus chronische obstruktive Lungenerkrankung (COPD), zystische Fibrose, chronische Bronchitis, Lungenfibrose, akutes Atemwegssyndrom, Lungenemphysem und Hämorrhagie, wobei die Aktivität der Elastase gehemmt oder inhibiert wird.

In einer bevorzugten Ausführungsform handelt es sich bei der Elastase um die humane neutrophile Elasfase (HNE).

Eine offenbarte Ausführungsform betrifft die Verwendung von Deuteriumoxid als Elastase-Inhibitor und insbesondere als Inhibitor der humanen neutrophilen Elastase (HNE) zur Prophylaxe und/oder Therapie von HNE-assozierten Erkrankungen.

Eine offenbarte Ausführungsform betrifft die Verwendung von Deuteriumoxid als Elastase-Inhibitor und insbesondere als Inhibitor der humanen neutrophilen Elastase (HNE) zur Herstellung eines Arzneimittels zur Prophylaxe und/oder Therapie von HNE-assozierten Erkrankungen.

Im Nachfolgenden umfasst der Begriff "erfindungsgemäße Verwendung" die Verwendung von D20 als Elastase-Inhibitor und insbesondere als HNE-Inhibitor sowie die Verwendung von D20 als Elastase-Inhibitor und insbesondere als HNE-Inhibitor zur Prophylaxe und/oder Therapie von HNE-assozierten Erkrankungen.

Die Begriffe "Prophylaxe und/oder Therapie" bezeichnen erfindungsgemäß jede für die Behandlung einer Elastase- bzw. HNE-assozierten Erkrankung geeignete Maßnahme, die entweder eine vorbeugende Behandlung einer solchen sich abzeichnenden Erkrankung bzw. deren sich abzeichnende Symptome oder die Vermeidung eines Wiederausbrechens einer solchen Erkrankung, beispielsweise nach einem abgeschlossenen Behandlungszeitraum darstellt (Prophylaxe) oder die Behandlung der Symptome einer solchen bereits ausgebrochenen Erkrankung darstellt (Therapie).

Unter "Elastase-assozierten Erkrankungen" bzw. "HNE-assozierten Erkrankungen" ist ein erfindungsgemäß ein pathologisches Erscheinungsbild zu verstehen, dass durch eine unkontrollierte oder unspezifische enzymatische Reaktion oder Aktivität einer Elastase bzw. der humanen neutrophilen Elastase (HNE) handelt, wobei die Reaktion oder Aktivität des Enzyms insbesondere in zu hohem Maß erfolgt.

Ein "Inhibitor" im Sinne der Erfindung bezeichnet eine Substanz, welche die enzymatische Reaktion oder Aktivität einer Elastase und insbesondere des Enzyms humane neutrophile Elastase (HNE) hemmt, verzögert oder inhibiert. Eine Hemmung oder Inhibierung der HNE ist vorzugsweise reversibel. Die Begriffe "Substanz", "Verbindung", "Molekül" und "Wirkstoff" werden im Sinne der Erfindung synonym verwendet.

Eine bevorzugte Ausführungsform der vorliegenden Erfindung betrifft folglich die erfindungsgemäße Verwendung von Deuteriumoxid, wobei die Aktivität der Elastase und insbesondere der humanen neutrophilen Elastase (HNE) gehemmt oder inhibiert wird.

Unter dem Begriff "hemmen" bzw. "Hemmung" gemäß der Erfindung ist zu verstehen, dass die enzymatische Aktivität der Elastase und insbesondere der humanen neutophilen Elastase (HNE) verlangsamt (verzögert) und/oder herabgesetzt wird, bevorzugt bis zu ca. 5%, vorzugsweise bis zu ca. 10%, ebenfalls bevorzugt bis zu ca. 20%, stärker bevorzugt bis zu ca. 30%, ebenfalls stärker bevorzugt bis zu ca. 40%, noch stärker bevorzugt bis zu ca. 50%, am stärksten bevorzugt bis zu ca. 60% gegenüber der enzymatischen Aktivität der Elastase und insbesondere von HNE ohne die Verabreichung von D20.

Der Begriff "inhibieren" oder "Inhibierung" gemäß der Erfindung bedeutet, dass die enzymatische Aktivität der Elastase und insbesondere der humanen neutophilen Elastase (HNE) verlangsamt (verzögert) und/oder herabgesetzt wird, bevorzugt über 50%, ebenfalls bevorzugt bis zu ca. 60%, weiterhin bevorzugt bis zu ca. 65%, vorzugsweise bis zu ca. 70%, ebenfalls bevorzugt bis zu ca. 80%, stärker bevorzugt bis zu ca. 90%, ebenfalls stärker bevorzugt bis zu ca. 95%, noch stärker bevorzugt bis zu ca. 98%, am stärksten bevorzugt um bis zu 100% gegenüber der enzymatischen Aktivität der Elastase und insbesondere von HNE ohne Verabreichung von D20.

Eine offenbarte Ausführungsform betrifft die Verwendung von D20 zur Prophylaxe und/oder Therapie von Elastaseassoziierten und insbesondere HNE-assozierten Erkrankungen, bei denen es sich um Entzündungserkrankungen, Lungenerkrankungen, Herzerkrankungen und/oder Kreislauferkrankungen und/oder allergische Erkrankungen handelt.

Bevorzugt handelt es sich bei den offenbarten Entzündungserkrankungen um eine Entzündung der Haut, der Nasenschleimhaut, der Mundschleimhaut, insbesondere aphthöse Erkrankungen der Mundschleimhaut, der Augenbindehaut der Nasennebenhöhlen oder Heuschnupfen, Asthma, cutane Vaskularitis, Lungenvaskularitis, Peritonitis oder septischer Schock ist.

Bevorzugt handelt es sich bei den offenbarten Entzündungserkrankungen um eine Entzündung der Haut, ausgewählt aus der Gruppe, bestehend aus neutrophilen Dermatosen, wie palmoplantar pustolosis, subcorneal pustolosis (Sneddon-Wilkinson's Krankheit), autoimmune bullöse Dermatose, Pemphigoid, wie bullöses Pemphigoid, pemphigoid vulgaris, Pemphigoid vegetans und Pemphigoid foliaceus.

Bevorzugt handelt es sich bei den Lungenerkrankungen im Sinne der vorliegenden Erfindung um eine Lungenerkrankung, ausgewählt aus der Gruppe, bestehend aus chronische obstruktive Lungenerkrankung (COPD), zystische Fibrose, chronische Bronchitis, Lungenfibrose, akutes Atemwegssyndrom, Lungenemphysem und Hämorrhagie.

Bevorzugt handelt es sich bei den offenbarten Herzerkrankungen und/oder Kreislauferkrankungen um eine Herzerkrankung und/oder Kreislauferkrankung, ausgewählt aus der Gruppe, bestehend aus Herzinfarkt, cerebrale Ischämie, Herzinsuffizienz und akutes Koronarsyndrom.

Bevorzugt handelt es sich bei den offenbarten allergischen Erkrankungen um eine allergische Erkrankung, ausgewählt aus der Gruppe, bestehend aus Hausstauballergie, Milbenallergie, Pflanzenpollenallergie und allergisches Asthma.

Die wirksame Prophylaxe und/oder Therapie von Elastase- und insbesondere HNE-assoziierten Erkrankungen kann insbesondere durch Verabreichung eines pharmazeutischen Wirkstoffes, genauer eines Inhibitors, erzielt werden, der unter Berücksichtigung der Art seiner Verabreichung, vorzugsweise alle der nachstehenden Eigenschaften aufweist:
a) bei topisch zu verabreichenden Elastase-Inhibitoren und insbesondere HNE-Inhibitoren: eine lokale Anwendbarkeit durch topische Verabreichung über einen beliebig langen Zeitraum und mit hoher perkutaner Transportkinetik des Wirkstoffs (Ensprechendes gilt für die parenterale Verabreichung);
b) bei durch ein Aerosol zu verabreichende Elastase-Inhibitoren und insbesondere HNE-Inhibitoren: Transportierbarkeit in die Lunge oder Nase in jeder gewünschten Partikelgröße zur willkürlichen bzw. selektiven Steuerung des Wirkstoffs (Elastase- bzw. HNE-Inhibitors) innerhalb des Lungengewebes;
c) eine weitgehend homogene Wirkstoffverteilung im Bereich des Wirkortes sowie die Vermeidung von lokalen Überkonzentrationen;
d) eine bevorzugte Anreicherung des Elastase-Inhibitors und insbesondere des HNE-Inhibitors in den betroffenen Körpergebietes, möglichst verbunden mit einer Retardierung des Transportes in den Blutkreislauf, d.h. in die Blutgefäße;
e) eine inhibitorische Wirkung, d.h. eine die Aktivität der Elastase und insbesondere der HNE hemmende bzw. inhibierende Wirkung im betroffenen Körpergebiet;
f) weitgehende Toleranz des Elastase-Inhibitors und insbesondere HNE-Inhibitors in dem umliegenden nicht pathologisch betroffenen Gewebe sowie den Blutkreislauf zur Vermeidung von Nebenwirkungen, insbesondere im Hinblick auf Immunreaktionen.

D20 als Wirkstoff bzw. Elastase-Inhibitor und insbesondere HNE-Inhibitor weist gegenüber bekannten Elastase-Inhibitoren und insbesondere HNE-Inhibitoren zur Behandlung von Elastase- bzw. HNE-assoziierten Erkrankungen erhebliche Vorteile auf, vor allem durch seine folgende Eigenschaften:
1) durch die Möglichkeit, D20 topisch auf die Haut zu applizieren, kann wegen des hohen kutanen, perkutanen und intrakutanen Transports von D20 in die Haut eine zur therapeutischen Wirksamkeit ausreichend hohe Konzentration (von mehr als 20% bezogen auf den Gesamtwassergehalt einer Zelle) von D20 in der Epidermis bzw. Dermis der Haut erreicht werden, ohne dass andere Organe des Körpers ähnlich hohen Konzentrationen von D20 ausgesetzt werden, wie es bei der systemischen Verabreichung erfolgen kann. Damit ist ein Entscheidendes, im Stand der Technik diskutiertes, Problem zur Erreichung von therapeutisch wirksamen D20-Konzentrationen am Wirkort (mehr als 20% D20 bezogen auf den Gesamtwassergehalt der Zelle) ohne starke Nebenwirkungen für andere Organe oder gesundes umliegendes Hautgewebe gelöst. Grundlage hierfür ist der gerichtete Transport von D20 durch das Stratum Corneum der Haut bis zur Epidermis bzw Dermis;
2) der Aggregatzustand von D20 kann bei einer topischen Verabreichung flüssig, gasförmig oder fest sein. Der Transport in die Haut kann durch direkten Kontakt der D20 bzw. einer D20-enthaltenden Formulierung mit der Haut und indirekt durch Diffusion über eine zwischengelagerte Schicht (z.B. Luft, poröse Membran, polymeres Netzwerk) erfolgen;
3) für die Verabreichung von reinem flüssigen D20 allein (reines D20), ist auszuführen, dass D20 gegenüber allen anderen flüssigen pharmazeutischen Wirkstoffen einen einzigartigen Vorteil hat. Es kann wie normales Wasser (H2O) in die Haut transportiert werden und durch die Stärke und Richtung des osmotischen Gradienten und einer Manipulation dieser beiden Größen kann weiterhin die Eindringtiefe von D20 in die Haut an die therapeutische Zielsetzung angepasst werden;
4) für die Verabreichung von D2O als Aerosol, ist auszuführen, dass die Oberflächenspannung von D2O der von H2O nahezu identisch und die Erzeugung von D2O-Aerosolen mit gezielt steuerbaren Partikelgrößen im Bereich 0,1 bis 10 µm erlaubt. Hierzu können zum Beispiel, aber nicht ausschließlich, etablierte Verfahren der mechanischen Zerstäubungstechnik verwendet werden. Durch die gezielte Wahl der Partikelgröße können D20-Aerosole mittels Inhalation in praktisch jeden Bereich der Lunge geschleust werden. Dort würden sie sich zunächst in den Alveolen anreichern und anschließend als Molekülcluster oder D20-Einzelmoleküle über die Barriere in das Lungengewebe transportiert werden. Damit ist durch die Größenwahl der D20-Partikel eine gezielte Einbringung (Optimierung des Wirkortes) des Wirkstoffs D20 für bestimmte Lungenbereiche möglich.
5) für die Verabreichung von D20 als Aerosol verabreicht wird, ist weiterhin auszuführen, dass D20-Aerosole gegenüber allen anderen flüssigen aerosolisierbaren pharmazeutischen Wirkstoffen einen einzigartigen Vorteil haben. Während in allen anderen Fällen Hilfsstoffe zugesetzt werden müssen, um den pharmazeutischen Wirkstoff stabil über das Aerosol in die Lunge zu transportieren, ist dies bei D20 nicht notwendig, da es als reines Molekül ohne Zusätze bereits optimal aerosolisierbar ist. Die Wirksamkeit in der Lunge beeinträchtigende Entmischungen, wie sie bei sonstigen Stoffgemischen (Wirkstoff/Hilfsstoff) im Verlauf der Aerosolisierung auftreten können sowie durch die Hilfsstoffe verursachte Nebenwirkungen sind damit ausgeschlossen. Unter "aerolisierbar" bzw. "Aerosolisierbarkeit" soll die grundsätzliche Möglichkeit verstanden werden, einen Stoff mittels Methoden, die Stand der Technik sind, in ein Aerosol mit kontrollierbarer Partikelgöße zu überführen.

Erfindungsgemäß wird nicht nur die Wirkung von D20 als Elastase-Inhibitor und insbesondere HNE-Inhibitor gezeigt, sondern auch, dass die Verabreichung einer Kombination aus D20 mit einem weiteren pharmazeutischen Wirkstoff, vorzugsweise einem weiteren Protease-Inhibitor, bevorzugt einem Serinprotease-Inhibitor, diese Wirkung noch verstärken kann. Ebenfalls kann zusätzlich oder anstelle eines weiteren pharmazeutischen Wirkstoffs die Verwendung von D20 zusammen mit einem weiteren nicht-pharmazeutischen Wirkstoff erfolgen.

Eine weiterhin bevorzugte Ausführungsform betrifft die erfindungsgemäße Verwendung von Deuteriumoxid, wobei Deuteriumoxid in Kombination mit mindestens einem weiteren pharmazeutischen Wirkstoff und/oder mindestens einem weiteren nicht-pharmazeutischen Wirkstoff verwendet wird. Eine solche Kombination aus D20 und mindestens einem weiteren pharmazeutischen Wirkstoff und/oder mindestens einem weiteren nicht-pharmazeutischen Wirkstoff wird nachfolgend als "erfindungsgemäße Kombination" bezeichnet.

Sämtliche in dieser Beschreibung offenbarten erfindungsgemäßen Verwendungen und Verabreichungen bzw. Verabreichungswege von D20 sind ebenfalls auf eine erfindungsgemäße Kombination uneingeschränkt anwendbar, sofern nichts Gegenteiliges angezeigt ist.

Der Begriff "pharmazeutischer" Wirkstoff bezeichnet im Sinne der vorliegenden Erfindung jede beliebige anorganische oder organische Substanz, der eine pharmakologische Wirkung zugeschrieben wird. Als pharmazeutischer Wirkstoff gemäß der vorliegenden Erfindung ist auch D20 sowie andere Elastase- und insbesondere HNE-Inhibitoren anzusehen.

Der Begriff "nicht-pharmazeutischer" Wirkstoff bezeichnet im Sinne der Erfindung jede pharmakologisch verträgliche und therapeutisch sinnvolle Substanz, die kein pharmazeutischer Wirkstoff ist, jedoch zusammen mit dem pharmazeutischen Wirkstoff in der pharmazeutischen Zusammensetzung formuliert werden kann, um qualitative Eigenschaften der pharmazeutischen Zusammensetzung zu beeinflussen, insbesondere zu verbessern. Bevorzugt entfalten die nicht-pharmazeutischen Wirkstoffe keine oder im Hinblick auf die beabsichtigte Therapie keine nennenswerte oder zumindest keine unerwünschte pharmakologische Wirkung.

Die strategischen Ziele dieser Beimischungen von weiteren pharmazeutischen und/oder nicht-pharmazeutischen Wirkstoffen liegen in der Verstärkung der HNEinhibitorischen Wirkung und/oder der Verbesserung der Verträglichkeit von D20.

Die Konzentration von erfindungsgemäß neben D20 als pharmazeutischem Wirkstoff verwendeten weiteren pharmazeutischen Wirkstoffen bezogen auf die Gesamtlösung einer erfindungsgemäßen Kombination, liegt im Bereich von mindestens 10⁻⁸ M bis mindestens 5 • 10⁻² M, bevorzugt von mindestens 10⁻⁷ M bis 10⁻³ M, am stärksten bevorzugt von mindestens 10⁻⁶ M bis mindestens 10⁻² M. Ein insbesondere bevorzugter Konzentrationsbereich liegt im Bereich von mindestens 10⁻⁹ M bis mindestens 10⁻² M. Nachfolgend werden erfindungsgemäß bevorzugte weitere (zusätzliche) pharmazeutische und nicht-pharmazeutische Wirkstoffe und deren Wirkung aufgeführt, wobei die vorliegende Erfindung nicht hierauf beschränkt ist:
Geeignete pharmazeutische Wirkstoffe sind insbesondere: Sulfonamide, Antibiotika (insbesondere Penizillin), Kortikoide, .Alkylfluorophosphate, Chloromethylketone, Sulfonylfluoride, Trifluoromethylketone, Borsäureester, Aldehyde, kurzkettige Peptide (insbesondere Peptide mit weniger als 10 Aminosäuren), Cytostatika, Chemotherapeutika, synthetische und pflanzliche Wirkstoffe mit entzündungshemmender Wirkung.

Geeignete nicht-pharmazeutische Wirkstoffe sind beispielsweise pharmazeutisch verträgliche anorganische oder organische Säuren oder Basen, Polymere, Copolymere, Block-Copolymere, Einfachzucker, Mehrfachzucker, ionische und nicht-ionische Tenside oder Lipiden, pharmakologisch unbedenkliche Salze, beispielsweise Natriumchlorid, Geschmackstoffe, Vitamine, z.B. Vitamin A oder Vitamin E, Tocopherole oder ähnliche im menschlichen Organismus vorkommende Vitamine oder Provitamine, Antioxidantien, wie beispielsweise Ascorbinsäure, sowie Stabilisatoren und/oder Konservierungsstoffe zum Verlängern der Verwendungs- und Aufbewahrungsdauer eines pharmazeutischen Wirkstoffes oder Formulierung und sonstige im Stand der Technik bekannte übliche nicht-pharmazeutische Wirkstoffe bzw. Hilfs- und Zusatzstoffe, sowie Mischungen hiervon. Weitere erfindungsgemäß bevorzugte nicht-pharmazeutische Wirkstoffe sind insbesondere alle zur Bildung wässriger Gele befähigten Stoffe, wie z.B. natürliche oder synthetische wasserlösliche Polymere, die Netzwerke ausbilden können.

Erfindungsgemäß werden bevorzugte weitere (zusätzliche) nicht-pharmazeutische Wirkstoffe und deren Wirkung aufgeführt, wobei die vorliegende Erfindung nicht auf diese beschränkt ist:
- Wasserlösliche Hilfs- und Zusatzstoffe
   Durch Zugabe von wasserlöslichen Hilfs- und Zusatzstoffen, wie z.B. pharmazeutisch verträglichen anorganischen oder organischen Säuren, Basen, Salzen und/oder Puffersubstanzen zur Einstellung des pH Wertes, kann die physiologische Verträglichkeit des D20 in der Lunge verbessert werden. Beispiele für bevorzugte anorganische Säuren sind Salzsäure, Bromwasserstoffsäure, Salpetersäure, Schwefelsäure und Phosphorsäure, wobei insbesondere Salzsäure und Schwefelsäure bevorzugt sind. Beispiele für besonders geeignete organische Säuren sind Äpfelsäure, Weinsäure, Maleinsäure, Bernsteinsäure, Essigsäure, Ameisensäure und Propionsäure und insbesondere bevorzugt Ascorbinsäure, Fumarsäure und Zitronensäure. Gegebenenfalls können auch Mischungen der genannten Säuren eingesetzt werden, insbesondere von Säuren, die neben ihren Säuerungseigenschaften auch andere Eigenschaften, z.B. in Verwendung als Geschmackstoffe oder Antioxidantien, besitzen, wie beispielsweise Zitronensäure oder Ascorbinsäure. Beispiele für pharmazeutisch verträgliche Basen sind Alkalihydroxide, Alkalicarbonate und Alkaliionen, bevorzugt Natrium. Mischungen dieser Substanzen können insbesondere zur Einstellung und Pufferung des pH Wertes benutzt werden, besonders bevorzugt sind hierbei Kaliumhydrogenphosphat und Di-Kaliumhydrogenphosphat sowie Natriumhydrogenphosphat und Di-Natriumhydrogenphosphat. Bevorzugte Puffersubstanzen im Sinne der Erfindung sind weiterhin PBS, HEPES, TRIS, MOPS sowie weitere physiologisch verträgliche Substanzen mit einem pK Wert im Bereich 5,0 bis 9,0.
   Die Konzentration dieser Substanzen, bezogen auf die Gesamtlösung, liegt vozugsweise im Bereich von Mikromolar bis Millimolar, besonders bevorzugt im Bereich 1- 100 mM.
- Wasserlöslichen, nicht-zytotoxischen Molekülen
   Durch Zugabe von wasserlöslichen, nicht-zytotoxischen Molekülen, wie z.B. bestimmten Polymeren (z.B., nicht hierauf beschränkt, Dextran, Polyethylenglykol, Zellulose, Hylaronsäure), Copolymeren und BlockCopolymeren kann durch deren hohe Wasserbindungskapazität eine zusätzliche Verzögerung (Retardierung) des Übergangs des D20 in den Blutkreilsauf erreicht werden. Für den Fall der erfindungsgemäßen Verwendung von D20 als Aerosol, kann durch die Fähigkeit der Polymere, die Dichte und/oder Viskosität von D20 verändern, darüber hinaus die Stabilität der D20-Partikel und sowie ihr Transport in die Alveolen der Lunge verändert und verbessert bzw. optimiert werden.
   Die Konzentration dieser Substanzen, bezogen auf die Gesamtlösung, liegt im Bereich von Millimolar bis Molar, bevorzugt im Bereich 1- 500 mM.
- Wasserlöslichen, nicht-Polymere Molekülen
   Durch Zusatz von wasserlöslichen, nicht-polymeren Molekülen, welche die Dichte und/oder Viskosität von D20 verändern, beispielsweise, aber nicht hierauf beschränkt, Einfachzucker und Mehrfachzucker, insbesondere Glukose, Sacharose, Dextrose, Maltose, Stärke und Zellulose, kann der Transport von D2O verändert und verbessert bzw. optimiert - und im Fall der erfindungsgemäßen Verwendung von D20 als Aerosol die Stabilität der D20-Partikel in den Alveolen der Lunge verändert und verbessert bzw. optimiert werden.
   Die Konzentration dieser Substanzen, bezogen auf die Gesamtlösung, liegt vorzugsweise im Bereich von Millimolar bis Molar, besonders bevorzugt im Bereich 1,0 mM bis 1,5 M..
- D2O-Grenzflächenspannungs-verändernde Substanzen
   Durch Zugabe von Substanzen, welche die Grenzflächenspannung von D20 verändern, beispielsweise, aber nicht hierauf beschränkt, ionische und nicht-ionische Tensiden oder Lipiden, insbesondere einer Mischung aus Tensiden und Lipiden, die in der Literatur summarisch als Lungensurfactants bezeichnet werden, kann die physikalisch-chemische Beschaffenheit, insbesondere die Aggregationsfähigkeit von D20-Partikel, z.B. Aerosol, verändert werden. Dies kann, beispielsweise in dem Fall der erfindungsgemäßen Verwendung von D20 als Aerosol, zu einer Veränderung der Stabilität und des Transportes der Partikel ins Lungengewebe genutzt werden. Darüber hinaus können derartige Moleküle in Kombination mit in D20 schwer löslichen pharmazeutischen Wirkstoffen die Funktion eines Lösungsvermittlers übernehmen und damit unter anderem, aber nicht herauf beschränkt, den Transport höherer Mengen
- pharmazeutischen Wirkstoffs pro Dosis, z.B. pro Aerosolpartikel, ermöglichen.
   Die Konzentration dieser Substanzen, bezogen auf die Gesamtlösung, liegt vorzugsweise im Bereich von Mikomolar bis Millimolar, besonders bevorzugt im Bereich 1- 500 mM.

Das erfindungsgemäß verwendete D20 liegt bevorzugt als Flüssigkeit vor. Vorzugsweise liegt das D20 in einer Lösung, bevorzugt mit H2O (Wasser) als Lösungsmittel, vor und wird hierin auch als "D2O/H2O-Lösung", wenn H2O enthalten ist, bezeichnet, oder als "D2O-Lösung" oder "reines D20", wenn kein H2O enthalten ist, bezeichnet. Reines D20 enthält D20 vorzugsweise in einem Konzentrationsbereich von 98,1 bis 100%, bevorzugt von 98,5 bis 99,9%, besonders bevorzugt von 99,7% bezogen auf den Gesamtwassergehalt der Lösung. Eine erfindungsgemäße D2O/H2O-Lösung enthält D20 vorzugsweise in einem Konzentrationsbereich von 1 bis 98%, bevorzugt von 5 bis 95%, weiterhin bevorzugt von 10 bis 90%, ebenfalls bevorzugt von 15 bis 80%, stärker bevorzugt von 20 bis 70%, ebenfalls stärker bevorzugt von 30 bis 60%, am stärksten bevorzugt von 40 bis 50%, wobei sich diese Angaben auf den Gesamtwassergehalt der Mischung aus D2O und H2O beziehen.

Die Herstellung einer erfindungsgemäßen D2O/H2O-Lösung, D20-Lösung und analog hierzu einer erfindungsgemäßen Kombination erfolgt beispielsweise durch Mischen der Komponenten, insbesondere von D2O, ggf. H2O sowie ggf. des mindestens einen weiteren pharmazeutischen und/oder nicht-pharmazeutischen Wirkstoffs. Weiterhin kann ggf. ein Lösungsmittel, wie nachfolgend beschrieben, durch Mischen hinzugefügt werden. Vorzugsweise erfolgt die Beimischung von H2O bzw. des mindestens einen weiteren pharmazeutischen und/oder nicht-pharmazeutischen Wirkstoffs bzw. des Lösungsmittels zu D20 im flüssigen Aggregatzustand. Die Herstellung kann jedoch durch jedes beliebige geeignete Verfahren erreicht werden

Sämtliche in dieser Beschreibung offenbarten erfindungsgemäßen Verwendungen und Verabreichungen von D20 sind sowohl auf D2O/H2O-Lösungen sowie D20-Lösungen uneingeschränkt anwendbar, sofern nichts Gegenteiliges angezeigt ist. Ebenfalls finden erfindungsgemäße Verwendungen von D2O/H2O-Lösungen sowie D20-Lösungen auch Anwendung auf die erfindungsgemäßen Kombinationen, Schichtsysteme, Pflaster und Bandage, Formulierungen und Aerosole uneingeschränkt Anwendung sofern nichts Gegenteiliges angezeigt ist.

Die erfindungsgemäße Verwendung von D20 kann, wie nachfolgend näher beschrieben, ebenfalls als Aerosol, Dampf oder Formulierung, insbesondere als Creme, Salbe, Gel oder Hydrogel erfolgen.

In einer offenbarten Ausführungsform ist der mindestens eine weitere pharmazeutische Wirkstoff bzw. weitere nicht-pharmazeutische Wirkstoff an D20 gebunden. "gebunden" im Sinn der vorliegenden Erfindung bedeutet, dass der pharmazeutische bzw. nicht-pharmazeutische Wirkstoff von dem D20 hydratisiert wird.

In weiteren offenbarten Ausführungsformen ist das D20 bzw. die erfindungsgemäße Kombination in einem geeigneten Lösungsmittel enthalten. Ein offenbartes Lösungsmittel kann ein anorganisches oder organisches Lösungsmittel sein. Geeignete Lösungsmittel der vorliegenden Erfindung sollten vorzugsweise von dem Organismus (insbesondere Säugetier), dem der Wirkstoff mit Lösungsmittel verabreicht wird, physiologisch gut verträglich sein, d.h. keine Nebenwirkungen, z.B. toxische Nebenwirkungen, auslösen. Ein insbesondere offenbartes Lösungsmittel ist destilliertes Wasser. Ebenfalls offenbart sind Ethanol-Wasser-Mischungen; hierbei liegt der prozentuale Massenanteil von Ethanol in diesen Mischungen bevorzugt im Bereich zwischen 5 % und 99 % Ethanol, ebenfalls bevorzugt im Bereich von 10 % bis 96 % Ethanol, stärker bevorzugt zwischen 50 % und 92 %, am stärksten bevorzugt zwischen 69 % und 91 % Ethanol.

Die Verabreichung von D20 kann erfindungsgemäß topisch, transdermal, nasal, rectal, parenteral, über eine Perfusion, über ein Endoskop oder als Aerosol oder Trockenpulverformulierung erfolgen.

Topische und transdermale Verabreichungen erfolgen durch Auftragen von D20 auf die Haut, vorzugsweise als D20-haltige Flüssigkeit (D20-Lösung, D2O/H2O-Lösung), Gas (Aerosol oder Dampf), Formulierung, vorzugsweise als Salbe, Creme, Lotion oder Emulsion, oder als D20-haltiges Gels oder Hydrogels. Diese Verabreichungsformen können vorzugsweise über ein Pflaster oder eine Bandage erfolgen.

Eine nasale Verabreichung erfolgt vorzugsweise über ein D20-haltiges Pulver oder D20-haltige flüssige Formulierung, die in die Nase geträufelt oder geschnupft werden.

Eine rectale Verabreichung erfolgt vorzugsweise über ein D20-haltiges Zäpfchen oder über eine Injektion einer D20-haltigen flüssigen Formulierung.

Eine parenterale Verabreichung erfolgt bevorzugt als Injektion oder Infusion einer D20-Formulierung und umfasst beispielsweise intravenöse, intraartikulär, intraartielle, intralymphatische, subkutane, intrakutane, intrapulmonale, intraperitoneale, intrakardiale, intrathekale, intrapleurale, intravitreale Verabreichung.

Eine Verabreichung über eine Perfusion erfolgt erfindungsgemäß vorzugsweise bei Herz- und/oder Kreislauferkrankungen.

Eine Verabreichung über ein Endoskop erfolgt erfindungsgemäß vorzugsweise bei Lungenerkrankungen.

Die Verabreichung kann weiterhin inhalativ, beispielsweise als Aerosol oder endobronchial (über einen Tubus) erfolgen.

Eine erfindungsgemäß bevorzugte topische oder transdermale Verabreichung von D20 ist insbesondere vorteilhaft bei den beschriebenen Entzündungserkrankungen der Haut und bei allergischen Erkrankungen. Bei der erfindungsgemäß bevorzugten topischen oder transdermalen Verabreichung können lokal hohe, therapeutisch wirksame D2O-Konzentrationen auf der Haut eingesetzt werden und gleichzeitig die Belastungen des Systems (d.h. des Blutkreislaufs) und die Nebenwirkungen auf nicht zu behandelndes, gesundes Hautgewebe sowie Gewebe von anderen Organen (wie beispielsweise der Leber oder Niere, die durch eine hohe Konzentration von D20 von mehr als 20% D20, bezogen auf den Gesamtwassergehalt der Zelle, verursacht werden könnten), zu vermindern bzw. vollständig zu vermeiden.

Ein Transport von D20 von den Hautzellen in das System kann darüber hinaus mit Mitteln, die im Stand der Technik gut bekannt sind, verhindert oder eingeschränkt werden kann. Beispiele für diese Mittel sind u.a. die gezielte Manipulation des osmotischen Gradienten über die Haut (d.h. zwischen systemischen Teil und der Hautoberfläche) durch Verringerung des Wasserpotentials des topisch applizierten D20 mittels Substanzen, die geeignet sind, dieses Wasserpotential zu verändern, insbesondere physiologisch verträgliche Salze, wie Natriumchlorid, wasserlösliche Polymere und andere nicht-pharmazeutische Stoffe.

Die topische Verabreichung von D20 kann ebenfalls über ein Pflaster oder eine Bandage erfolgen. Erfindungsgemäß insbesondere offenbart ist daher die erfindungsgemäße Verwendung von D20, wobei D20 mit einem bzw. über ein Pflaster oder eine Bandage topisch appliziert wird.

Als "Pflaster" oder "Bandagen" im Sinne der Erfindung, sind alle auf der Haut durch mechanische oder chemische Wechselwirkung, Physisorption, Adhäsion oder andere physikalisch-chemische Prozesse fixierbare Vorrichtungen zu verstehen, welche dazu geeignet sind, einen ausgewählten Hautbereich okklusiv oder nicht-okklusiv über einen für die beabsichtigte Behandlung angemessen langen Zeitraum abzudecken und die Zuführung von D20 an die Haut zu ermöglichen und/oder unterstützen. Erfindungsgemäß anwendbare Pflaster und Bandagen als Applikationssysteme zur lokalen Freisetzung von Wirkstoffen auf der Haut (z.B. Wärmepflaster) sowie zur kontrollierten systemischen Freisetzung von Wirkstoffen (z.B. Opiat-Depotpflaster, Nitroglycerin-Depotpflaster) sind im Stand der Technik bekannt. Als "Depot-Pflaster" oder "Depot-Bandage" soll zusätzlich zu den oben beschriebenen Eigenschaften die Fähigkeit des Pflasters oder der Bandage zur Speicherung von D20 und dessen kontrollierte Abgabe an die Haut über einen Zeitraum von Tagen oder Wochen verstanden werden. Solche Depot-Pflaster bzw. Depot-Bandagen sind nachfolgend von den Begriffen Pflaster bzw. Bandage umfasst.

Insgesamt ist für jede kontrollierte Freisetzung von D20 an die Haut folgendes Problem zu beachten: die Freisetzung aus einer direkt auf die Haut aufgetragenen Flüssigkeit oder Formulierung als Salben, Creme oder Gel kann durch die Richtung des osmotischen Gradienten innerhalb der Haut von innen nach außen erschwert werden, da das D20 in der Flüssigkeit, Salbe, Creme oder in dem Gel unter Umständen ein niedrigeres Wasserpotential aufweist als das H2O in der Haut und in den darunter liegenden Gefäßen.

Eine offenbarte Ausführungsform der topischen Applikation von D20 ist es daher, durch gezielte Manipulation der osmotischen Verhältnisse im zu behandelnden Hautgebiet die Tiefe bzw. den Grad des Eindringens von D20 in die Haut zu regulieren und damit zu kontrollieren, bevorzugt bis zur Epidermis oder bis zur Dermis. Dies kann durch die gewählte Zusammensetzung einer applizierten erfindungsgemäßen Kombination erreicht werden, indem Substanzen zugefügt werden, welche in der Lage sind, die osmotischen Verhältnisse an der Oberfläche der Haut zu verändern.

Eine weitere Möglichkeit zum kontrollierten Eindringen von D20 in die Haut besteht in der Verwendung von einer oder mehreren Membran(en) oder Folie(n), welche die Passage von Wasser und Gasen ermöglicht/ermöglichen, von größeren Molekülen oder Partikeln (einschl. Bakterien, Viren, Einzeller) jedoch verhindert/verhindern. Beispiele für solche verwendbaren Membranen und Folien sind im Stand der Technik bekannt und haben zahlreiche Anwendungen, wie z.B. in Textilien unter dem Markennamen GORE Tex® oder in der Medizin sogenannte Biofilme oder atmungsaktive Pflaster wie z B. Tegaderm®.

Offenbart ist demnach die erfindungsgemäße Verwendung von D20, wobei das D20 topisch mit einem Pflaster oder einer Bandage appliziert wird, wobei das Pflaster oder die Bandage in Kombination mit mindestens einer Membran oder mindestens einer Folie verwendet wird. Vorzugsweise ist die mindestens eine Membran bzw. die mindestens eine Folie eine mikro- oder nanoporöse Membran bzw. Folie.

Eine beispielhaft offenbarte Anordnung für eine solche erfindungsgemäße topische Verwendung von D20 besteht aus folgenden Komponenten:
- einer vorstehend beschriebenen mikro- oder nanoporösen Membran oder Folie, welche direkt auf die Haut aufgebracht wird,
- gefolgt von einer D20-Schicht, welche das D20 enthält,
- ggf. gefolgt von einer sog. Okklusionsschicht welche eine Verdampfung des D20 nach außen verhindert oder reguliert und gleichzeitig einen mechanischen Schutz darstellt, welcher das Entweichen von D20 als Flüssigkeit verhindert und
- gefolgt von einem Pflaster oder einer Bandage.

Eine weitere beispielhafte Anordnung für eine solche erfindungsgemäße topische Verwendung von D20 besteht aus folgenden Komponenten:
- einer vorstehend beschriebenen mikro- oder nanoporösen Membran oder Folie, welche direkt auf die Haut aufgebracht wird,
- gefolgt von einer D20-Schicht, welche das D20 enthält,
- ggf. gefolgt von einer sog. Okklusionsschicht welche eine Verdampfung des D20 nach außen verhindert oder reguliert und gleichzeitig einen mechanischen Schutz darstellt, welcher das Entweichen von D20 als Flüssigkeit verhindert und
- gefolgt von einer weiteren vorstehend beschriebenen mikro- oder nanoporösen Membran oder Folie.

Bei Bedarf können selbstverständlich weitere D20-Schichten hinzugefügt werden, die D20 enthalten, und die von Membranen bzw. Folien separiert werden, wodurch die Depotwirkung oder der Übergang des D20 in die Haut verändert werden kann, beispielsweise die Freisetzungsmenge und/oder -dauer von D20. Der verwendete Begriff " "D2O-Schicht" bezieht sich auf flüssiges eine D20-Lösung, (reines D20), D2O/H2O-Lösung, erfindungsgemäße Kombination sowie auf eine erfindungsgemäße Formulierung von D20, insbesondere als Creme, Salbe, Gel oder Hydrogel.

Ebenfalls können vorzugsweise auch Schichten hinzugefügt werden, die chemische, elektrische oder thermische Eigenschaften aufweisen, welche geeignet sind, den Übergang des D20 in die Haut und/oder die Dauer seiner Freisetzung zu manipulieren. Beispiele hierfür sind Schichten, die geeignet sind, ein elektrisches, thermo-elektrisches, thermisches oder chemisches Potential (oder eine Kombination davon) über die darunter liegenden Schichten und die Haut aufzubauen und/oder aufrechtzuerhalten. Dies kann beispielsweise durch in die beschriebenen Membranen oder Folien eingebettete oder auf ihnen befindliche Elektroden erreicht werden, welche von außen mit Strom (Gleichspannung, Wechselspannung oder hochfrequente Ströme) versorgt werden oder die durch die gezielte Wahl des Elektrodenmaterials mit der D20 Schicht als Elektrolyt elektrochemische Potentiale erzeugen.

Die Gesamtheit solcher vorbeschriebenen D20-Schichten, Okkulsionsschichten, Schichten mit chemischen, elektrischen oder thermischen Eigenschaften, Membranen und Folien in jeder beliebigen, für den Verwendungszweck geeigneten Anzahl, Kombination und Anordnung, wird nachfolgend als "Schichtsystems" bezeichnet. Ein solches Schichtsystem wird vorzugsweise in Verbindung mit einem/einer vorstehend beschriebenen (Depot-) Pflaster oder (Depot-) Bandage verwendet werden.

Durch Variation von Morphologie (Porengröße, Membran- bzw. Foliendicke, Oberflächenrauheit und Oberflächenprofil) und Oberflächeneigenschaften (z.B. hydrophil oder hydrophob, chemischen Beschichtungen -kovalent gebunden oder adhäsiv gebunden-, funktionellen Gruppen, Bindung oder Einlagerung anorganischer oder organischer Substanzen) der direkt im Kontakt mit der Haut befindlichen Membran oder Folie kann der Übergang des D20 aus einem beschriebenen Pflaster, Bandage oder Schichtsystem in die Haut gezielt beeinflusst bzw. verändert werden.

Eine weitere Variation des Eintritts von D20 in die Haut ist durch die gezielte Verwendung von Klebstoffen möglich, welche zur mechanischen Befestigung des (Depot-) Pflasters oder der (Depot-) Bandage auf der Haut verwendet werden können, aber nicht zwingend notwendig sind. Die allgemein für topische Anwendungen von Pflastern und Bandagen verwendeten Klebstoffe weisen eher hydrophoben Charakter auf, welcher den Durchtritt von D20 durch die Klebstoffschicht verhindert kann. Durch Zumischen von Zusatzstoffen in die Klebstoffzubereitung kann eine Veränderung dieser Eigenschaften erreicht werden. Als solche "Zusatzstoffe" kommen organische und/oder anorganische Substanzen und Verbindungen in Betracht, welche in der Lage sind, die Permeationseigenschaften von D20 durch die Klebstoffschicht zu verändern. Beispiele für solche Substanzen sind u.a. Polymere, Co-Polymere, Blockpolymere, Block-Co-Polymere, Tenside, Peptide, Proteine, Nukleinsäuren, Sterole und Steroide.

In einer weiteren offenbarten Ausführungsform erfolgt die erfindungsgemäße Verwendung von D20, vorzugsweise mit einem Pflaster oder einer Bandage, topisch auf die Haut über eine Anordnung, die den Übergang von D20 in die Haut weitgehend oder ausschließlich über die Dampfphase ermöglicht. D.h. erfindungsgemäß als Flüssigkeit verwendetes D20 verdampft als molekulares D20 und kommt als Dampf mit der Haut in Kontakt. Die Konzentrationen des D20 entsprechen damit den oben beschriebenen Konzentrationen einer D20-enthaltenden Flüssigkeit. Dampfförmiges D20 hat den Vorteil eines besonders leichten Eindringens in die Haut. Um dieses Verdampfen zu bewirken, ist thermische Energie erforderlich, die entweder von der Haut selbst oder von einer externen Wärmequelle, z.B. bei Verwendung eines beschriebenen Pflasters oder Bandage oder oben beschriebenen Schichtsystems hierin eingebrachten elektrischen Heizung (z.B. Peletier-Heizung), bezogen werden kann. Zusätzlich zu dieser thermischen Energie kann durch gezielte Modifikationen, beispielsweise der Wahl der Morphologie (insbesondere Porengröße und Oberflächenbeschichtung), einer ersten, auf der Haut befindlichen oben beschriebenen Membran oder Folie des Schichtsystems erreicht werden, dass nur dampfförmiges D20 durch die Membran oder Folie bis zur Haut vordringen kann, flüssiges D20 hingegen zurückgehalten wird. Auch bei dieser Ausführungsform lassen sich durch die oben beschriebenen Veränderungen des Schichtsystems und entsprechenden Modifikationen an ggf. weiteren verwendeten Membranen oder Folien, die Menge und die Dauer der Freisetzung von D20 über die Dampfphase kontrolliert und/oder verändert werden.

Eine erfindungsgemäß bevorzugte Verabreichung von D20 als Aerosol ist insbesondere vorteilhaft bei den beschriebenen Lungenerkrankungen, Entzündungen der Nasennebenhöhlen, der Nasenschleimhaut, Heuschnupfen, Asthma und allergisches Asthma und den sonstigen allergischen Erkrankungen

Eine erfindungsgemäß bevorzugte Verabreichung von D20 als Aerosol erfolgt über die Inhalation von D20. Die Inhalation von Wirkstoffen über die Lunge eines Organismus, bevorzugt eines Säugetieres, ist eine seit Jahren bekannte und zunehmend angewendete Technik zur lokalen und systemischen Freisetzung dieser Substanzen. Sie basiert auf dem Transport von Partikeln im Größenbereich von einigen hundert µm bis zu wenigen nm im Luftstrom beim Einatmen, gefolgt von der Deposition der Partikel in den Lungenbläschen (Alveolen), von wo aus sie dann in das System eindringen und zum Wirkort innerhalb des Organismus transportiert werden. In manchen Fällen ist auch die Lunge selbst der Wirkort. Als Partikel werden erfindungsgemäß im folgenden alle im Größenbereich von 0,005 µm bis 100 µm liegenden Gebilde Moleküle oder Makromoleküle bezeichnet, ungeachtet ob sie einen festen oder flüssigen Aggregatzustand aufweisen. Die Partikel werden hierzu als Aerosol formuliert und von dem Patienten eingeatmet (inhaliert), vorzugsweise über geeignete Inhalatoren (auch Vernebler genannt).

Weiterhin offenbart ist daher ein D20-enthaltendes Aerosol.

Unter einem Aeorosol versteht man feste oder flüssige schwebende Partikel mit einem Durchmesser von ca. 0,0001 µm bis ca. 100 µm, in Gasen insbesondere Luft, wobei Zusammensetzung und Form der Aerosole sehr stark variieren kann. Aerosole lassen sich künstlich durch im Stand der Technik gut bekannte Dispersions- und Kondensationsverfahren herstellen. Sie können ohne Treibgas verwendet werden oder in Verbindung mit einem verflüssigten Druckgas als Treibgas in Sprühdosen verwendet werden. Aerosole werden (mit und ohne Treibgas) häufig auf dem Gebiet der Medizin für sog. Aerosoltherapien zum Transport von pharmazeutischen Wirkstoffen in die Lunge eingesetzt. Sofern im Nachfolgenden der Begriff Aerosol verwendet wird, bezieht sich dieser auf medizinische Aerosole. Die kleinsten pharmazeutisch wirksamen Partikel in Aerosolen sind beispielsweise Nukleinsäuren, Peptide oder Proteine, die größten Partikel beispielsweise Nebelpartikel. Häufig bestehen Aerosole aus Gemischen von Partikeln unterschiedlicher Partikelgrößen und verkörpern dadurch eine polydisperse Größenverteilung.

Die Herstellung erfindungsgemäß verwendeter Aerosole (mit und ohne Lösungsmittel) kann durch jedes beliebige im Stand der Technik bekannte Verfahren hergestellt werden. Es sind hierzu zahlreiche Standardverfahren bekannt.

Die Inhalation der Aerosole erfolgt oral oder nasal durch den zu behandelnden Organismus, insbesondere eines Säugetiers, und Ziege, vorzugsweise über einen Inhalator. Nach der Inhalation der Partikel in die Lunge weicht ein gewisser Anteil der Partikel von der Stromlinie des Aerosols ab und gelangt dabei in Kontakt mit der feuchten Oberfläche der Lufträume. Dieses Phänomen wird im Allgemeinen als Partikeldeposition bzw. Deposition bezeichnet und unterliegt drei physikalischen Mechanismen:
- Impaktion, beruhend auf der Massenträgheit des Aerosols, liegt bei Partikeln mit einem Durchmesser ab ca. 3 µm vor,
- Sedimentation, beruhend auf der Schwerkraft der Partikel, erfolgt bei Partikeln mit einem Durchmesser von ca. 0,5 bis 1 µm und
- Diffusion, beruhend auf der Brownschen Molekularbewegung, tritt bei Partikeln mit einem Durchmesser von ca. kleiner als 1 µm auf.

Die Wahl der Partikelgröße der zu transportierenden pharmazeutischen Wirkstoffe ist demnach ein entscheidender Faktor für deren Depositionsmechanismus in der Lunge.

Neuere Entwicklungen der Inhalationstechnik lassen sich in 2 Schwerpunkte unterteilen:
1.) die Optimierung von Freisetzungsmethoden im Sinne einer weitgehenden Kontrolle über die physikalischen Eigenschaften der zu inhalierenden Partikel und
2.) die Optimierung der zur Freisetzung benutzten Wirkstoffformulierungen im Sinne einer Verbesserung der kolloidalen Eigenschaften der Partikel für die Inhalation und ihrer systemischen Wirksamkeit.

Unter Punkt 1) lassen sich alle Entwicklungen von Zerstäubungs- bzw. Inhalationssystemen für Festkörper, Flüssigkeiten und Gase zusammenfassen. Diese konzentrieren sich auf die Bereiche mechanische Zerstäubung, Verdampfung usw. mit dem Ziel einer Kontrolle insbesondere der Partikelgröße und Partikelstabilität. Die Entwicklungen unter 2) umfassen Verbesserungen des Agglomerationsverhaltens der Partikel, ihrer Freisetzungs- und Transporteigenschaften in den Lungenbläschen (Alveolen) sowie der Stabilität der Wirkstoffe in den Partikeln.

Da die physikalisch-chemischen Eigenschaften der Partikel (inbesondere Größe, Stabilität, Löslichkeit in wässriger Umgebung, Oberflächenladung, Bindungseigenschaften, Keimbildungsverhalten, Grenzflächeneigenschaften) durch den pharmazeutischen Wirkstoff selbst mitbestimmt werden, ist für spezielle pharmazeutische Wirkstoffe und deren Formulierungen häufig noch eine individuelle Anpassung der Freisetzungsmethoden erforderlich. Das Ergebnis eines solchen oft langwierigen Anpassungsprozesses ist (angesichts der Vielzahl an zu berücksichtigenden Parametern) fast immer ein Kompromiss zwischen (i) den Inhalationseigenschaften, (ii) den Transporteigenschaften der Partikel in der Lunge bzw. im Lungenbläschen, (iii) der individuellen Inhalationstechnik des Patienten und (iv) der therapeutischen Wirksamkeit in der Lunge oder im System.

Insgesamt lässt sich folgendes zusammenfassen:
1.) Gasförmige Substanzen sind ideal geeignet für die Applikation über die Lunge (z.B. Narkosegase bei künstlicher Beatmung), da sie meist ohne Probleme durch die Lungenbarriere gelangen und praktisch unmittelbar systemisch verfügbar sind. Allerdings werden sie auch ebenso rasch wieder aus dem System entfernt, haben somit keine Depotwirkung und erlauben keine Optimierung des Wirkortes im System. Unter "Depotwirkung" ist hierbei die zeitlich verzögerte Freisetzung des pharmazeutischen Wirkstoffs im System und - unter Optimierung des Wirkortes - die überdurchschnittliche Anreicherung des pharmazeutischen Wirkstoffes im zu therapierenden Gewebe oder Organ verstanden werden.
2.) Flüssige Partikel können hingegen pharmazeutische Wirkstoffe, nicht-pharmazeutische Wirkstoffe, insbesondere Hilfsstoffe, gemeinsam transportieren und erlauben eine Depotwirkung und eine Optimierung des Wirkortes. Allerdings haben solche flüssigen Aerosole aus komplexen Stoffgemischen oft Stabilitätsprobleme, welche die Wirksamkeit des Wirkstoffs in der Lunge oder im System beeinträchtigen können.
   Feste Partikel sind den flüssigen Partikeln durch ihre hohe Stabilität überlegen und können mit den heutigen Freisetzungsmethoden (Zerstäubern bzw. Verneblern) über einen weiten Größenbereich hergestellt werden. Damit lassen sich, wie bei flüssigen Partikeln, auch die Eindringtiefen der Partikel in die Lunge gezielt steuern. Partikel im Größenbereich von 1,5 µm und weniger können praktisch jedes Lungengewebe erreichen, während größere Partikel hauptsächlich in die Bereiche der luftleitenden Atemwege (Bronchien) eindringen.

Ein möglicher Zielort bzw. Wirkort (Target) der mittels Inhalation übertragenen pharmazeutischen Wirkstoffe bzw. ihrer Formulierungen ist die Lunge selbst, insbesondere bei den bereits oben beschriebenen medizinischen Indikationen, für die eine Verabreichung von D20 als Aerosol geeignet ist.

Die Verabreichung eines erfindungsgemäß verwendbaren Aerosols erfolgt bevorzugt über einen Inhalator, auch Vernebler genannt. Als "Inhalator" für die vorliegende Erfindung ist jeder beliebige für medizinische Aerosole geeignete Standardinhalator verwendbar. Ein "Inhalator" kann ebenfalls für die Herstellung erfindungsgemäßer Aerosole verwendet werden. Die erfindungsgemäßen D20-Lösungen, D2O/H2O-Lösungen, erfindungemäßen Kombinationen werden dem Inhalator zugeführt, um aus hieraus die erfindungsgemäßen, bevorzugt treibgasfreien, Aerosole herzustellen. Der Inhalator versprüht hierzu ein definiertes Volumen der Formulierung unter Anwendung hoher Drücke durch kleine Düsen, um so ein inhalierbares erfindungsgemäßes Aerosol zu erzeugen. Besonders geeignet sind hierbei Inhalatoren, die eine kleine Menge einer flüssigen erfindungsgemäßen D20-Lösung in einer therapeutisch geeigneten Dosierung binnen weniger Sekunden in ein therapeutisch-inhalativ geeignetes Aerosol vernebeln können. Solche Inhalatoren sind insbesondere für eine treibgasfreie Verabreichung der erfindungsgemäßen Aerosole oder pharmazeutischen Zusammensetzungen geeignet. Ein derartiger Vernebler ist z.B. in den internationalen Patentanmeldungen WO 91/14468 und WO 97/12687 beschrieben. In einem solchen Vernebler wird eine Arzneimittellösung mittels hohen Drucks von bis zu 600 bar in ein medizinisches, für die Applikation an Atemwege und Lunge geeignetes, Aerosol überführt und versprüht. Zur Vernebelung einer solchen Lösung wird eine spezielle Düse verwendet, wie sie beispielsweise die WO 94/07607 oder die WO 99/16530 beschreibt.

Geeignete Inhalatoren für erfindungsgemäße Aerosole umfassen auch Treibgasgetriebene Inhalationsgeräte (bzw. Vernebler). Treibgase können hierbei beispielsweise FCKW oder HFKW sein. Diesbezüglich wird auf "Theorie und Praxis der Inhalationstherapie", Seiten 31 - 70, Arcis Verlag (2000) verwiesen, wo eine ausführliche Beschreibung verwendbarer Vernebler sowie Verfahren zu deren Anwendung offenbart ist/sind.

Weitere Beispiele für geeignete Inhalatoren sind pressluftgetriebene Düsenvernebler (z.B. PARI LC plus, PARI GmbH, Starnberg, Deutschland), Venturi-Düsenvernebler, Wasserdampfgetriebene Düsenvernebler oder Ultraschallvernebler (z.B. AeronebLab, Aerogen, Inc., Stierlin Court, Canada; eFLOW, PARI GmbH, Starnberg, Deutschland). Ebenfalls geeignet sind Inhalatoren mit einer Größe, die von dem Patienten (Mensch) mitgeführt werden können, z.B. der Respimat@, wie in WO 97/12687, beschrieben. Eine ausführliche Beschreibung geeigneter Inhalatoren findet sich auch in "Theorie und Praxis der Inhalationstherapie", Seiten 31- 70, Arcis Verlag (2000). Sämtliche in der Beschreibung der vorliegenden Erfindung aufgeführten Referenzen werden vollumfänglich in die vorliegende Erfindung eingeschlossen.

Eine weitere bevorzugte Verabreichungsform der vorliegenden Erfindung ist die Verabreichung von D20 über ein Endoskop (Bronchoskopie). Diese bevorzugte Verabreichung von D20 führt zu einer Erhöhung der in den Alveolen verfügbaren Menge von D20. Eine solche Erhöhung kann unter folgenden Bedingungen notwendig sein:
1.) die Prozesse des Ein- und Ausatmens begrenzen die den Alveolen durch Inhalation maximal zuführbare Menge von D20. Diese kann dadurch für bestimmte therapeutische Ziele unterhalb der als notwendig erachteten Menge liegen,
2.) durch Anhäufung von Zellen infolge von Fibrose oder Lungentumor können Lungenbereiche vom Gasaustausch bereits weitgehend ausgeschlossen sein, in diesem Fall würde D20, das als Aerosol verabreicht werden, nicht effektiv bis in die dort befindlichen Alveolen gelangen.

In diesen Fällen erfolgt erfindungsgemäß ein bevorzugter direkter Kontakt von D20 als Flüssigkeit mit Teilen der Lungenoberfläche durch Einfüllen von D20 in Teile der Lunge (z.B. mit Hilfe eines Endoskops). Unter "Endoskop" ist jedes geeignete Gerät zum Verabreichen von D20 an die Lunge eines Säugetieres.

Im Zusammenhang der vorliegenden Erfindung ist der Begriff "Endoskop" synonym mit dem Begriff "Bronchoskop" zu verstehen. Das Befüllen der Lunge mit Wasser bzw. mit wäßrigen Lösungen und dessen spätere Entfernung ist Stand der Technik bekannt und wird bei bestimmten Indikationen -meistens im Rahmen einer Bronchoskopie- zum Spülen der Lunge angewandt (Broncho-Alveoläre Lavage, BAL). Gemäß der vorliegenden Erfindung wird das D20, vorzugsweise über ein Endoskop, in Teile der Lunge so eingefüllt, dass die Alveolen in diesem Bereich weitestgehend mit D20 gefüllt sind. Anschließend wird das D20 für eine der Therapie angemessene Zeit, beispielsweise 2 bis maximal 36 Stunden in der Lunge belassen und dann wieder entfernt. Die D20-Applikation kann, wenn erforderlich, in zeitlichen Abständen über mehrere Monate, vorzugsweise 2 Monate, bevorzugt 3 Monate, stärker bevorzugt 6 Monate, noch stärker bevorzugt 12 Monate hinweg wiederholt werden. Der Vorteil dieses im Folgenden als D20-BAL bezeichneten Vorgehens ist die direkte Kontaktierung der Alveolenoberfläche mit D20 und damit das maximale Eindringen von D20.

Bevorzugte erfindungsgemäße Ausführungsformen der D20-BAL stellen die Verwendungen einer erfindungsgemäßen D20-Lösung dar mit nachfolgenden Ergänzungen bzw. Modifikationen dar, deren Wirkung beschrieben wird. Es versteht sich, dass diese Aufzählung nicht abschließend ist:
1.) Beimischung von H2O zu einer erfindungsgemäßen D20-Lösung. Hierdurch kann die für den Therapiezweck optimale Menge an D20 eingestellt werden,
2.) Verwendung einer erfindungsgemäßen D20-Lösung mit einer möglichst hohen D20 - Konzentration (bevorzugt D20 mit einer Isotopenreinheit von größer als 95%), die eine entsprechend hohe Anreicherung von D20 im ausgewählten erkrankten Lungenbereich bewirkt. Dieses Vorgehen kann kombiniert werden mit der Zugabe von geeigneten Molekülen zur D20 Lösung, welche die osmotischen Verhältnisse an der alveolaren Grenzfläche deutlich in eine Richtung verändern, ohne jedoch einen osmotischen Schock hervorzurufen. In diesem Fall würde die Mischung von zellulärem Wasser (aus dem Zytoplasma der alveolären Epithelschicht) mit dem alveolären D20 Wärme erzeugen, die direkt an der Grenzfläche freigesetzt würde.
3.) Zugabe von Substanzen, die geeignet sind, die osmotischen Verhältnisse zwischen der applizierten erfindungsgemäßen D2O-Lösung und den Alveolen zu verändern. Damit kann eine Steuerung des Eintritts von D2O in die Zellen sowie auch des Austritts von zellulärem H2O in die alveoläre D20-Lösung erfolgen.

Erfindungsgemäß kann das für das D2O-BAL verwendete D2O bevorzugt auch als erfindungsgemäße Kombination verwendet werden. Hierzu geeignete bevorzugte weitere pharmazeutische bzw. weitere nicht-pharmazeutische Wirkstoffe schließen die oben bereits ausführlich beschriebenen Wirkstoffe ein. Die Konzentrationen von D20 sowie der pharmazeutischen bzw. nicht-pharmazeutischen Wirkstoffe beziehen sich hierbei auf die bereits vorgenannten Konzentrationsangaben.

Eine weitere bevorzugte Ausführungsform der vorliegenden Erfindung betrifft die Verwendung von Deuteriumoxid, wobei Deuteriumoxid als Formulierung verabreicht wird.

Weiterhin offenbart ist daher eine D20-enthaltende Formulierung.

Bevorzugt ist eine solche erfindungsgemäße Formulierung eine Salbe, eine Creme, eine Lotion, eine Emulsion oder ein Gel oder ein Hydrogel ist.

Unter einer "Salbe" gemäß der vorliegenden Erfindung ist eine äußerlich zu verwendende Arzneimittelzubereitung aus einer Grundmasse schmierfähiger Stoffe, wie Vaseline, zu der die eigentlichen pharmazeutischen und/oder nicht pharmazeutische Wirkstoffe zugegeben werden, beispielsweise durch Mischen.

Unter einer "Creme" im Sinne der vorliegenden Erfindung ist eine erfindungsgemäße Salbe zu verstehen, die zusätzlich weitere Bestandteile enthalten kann, wie kosmetische Wirkstoffe, z.B. Duftstoffe, Farbstoffe und/oder Emulgatoren, z.B. Lecithin. Von einer Creme wird im allgemeinen eine Lotion unterschieden, wobei diese Unterscheidung meistens abhängig von dem Grad der Viskosität gemacht wird. Erfindungsgemäß ist unter einer Creme auch eine Lotion zu verstehen.

Unter einer "Emulsion" im Sinne der vorliegenden Erfindung ist eine Makro- oder Mikroemulsion zu verstehen, entweder auf Wasser-in -Öl oder auf Öl-in-Wasser Basis.

Ein "Gel" im Sinne der vorliegenden Erfindung ist die Lösung einer makromolekularen Substanz, z. B. Agarose, Acrylsäure, Alginsäure, Polysiloxane oder Acrylamid, deren Konzentration so hoch ist, dass sich die gelösten Makromoleküle unter geeigneten Bedingungen und ggf. unter Zugabe weiterer Substanzen (z. b. Salze, Säuren, Füllstoffe, Puffersubstanzen) zu einem schwammartigen, dreidimensionalen Gerüst verbinden, in dessen Hohlräumen sich eine Flüssigkeit befindet. Dadurch haben Gele eine relativ feste Konsistenz. Die Viskosität liegt zwischen flüssig und fest. Eine solche Flüssigkeit ist bevorzugt reines D20 oder eine Mischung aus D2O und H2O.

Als "Hydrogel" im Sinne der Erfindung wird ein Gel bezeichnet, welches durch besonders hohe Aufnahmekapazität von Wasser gekennzeichnet ist, im Sinne der Erfindung besteht es bevorzugt zu 20-99%, stärker bevorzugt zu 70-99%, und besonders bevorzugt zu 80-99%, aus Wasser, ohne jedoch die rheologischen Eigenschaften einer klassischen Flüssigkeit zu zeigen. In einer besonders bevorzugten Ausführungsform ist das Hydrogel transparent-durchsichtig und gleichzeitig streichfähig, ohne das seine Morphologie und Integrität durch das verstreichen des Gels beeinträchtigt wird.

Die Herstellung einer erfindungsgemäß verwendbaren Formulierung, insbesondere einer Salbe, Creme Lotion, Emulsion, oder einem Gel oder Hydrogel, ist exemplarisch in den Beispielen beschrieben. Sofern eine solche Formulierung weitere pharmazeutische und/oder nicht-pharmazeutische Wirkstoffe enthalten, werden diese vorzugsweise durch Mischen der Formulierung hinzugefügt. Sie kann jedoch nach jedem beliebigen im Stand der Technik bekannten Standardverfahren erfolgen. Dem Fachmann sind solche Verfahren und ebenfalls die zu wählenden Konzentrationen der zu verwendenden Komponenten bzw. Substanzen bekannt.

Die Konzentrationen von D20 in einer erfindungsgemäß verwendbaren Formulierung liegen vorzugsweise in folgenden Bereichen:
- für eine Creme oder Salbe bevorzugt im Bereich von 0,1 bis 95 Gew.-%, bevorzugt von 5 bis 85 Gew.-%, ebenfalls bevorzugt von 10 bis 80 Gew.-%, besonders bevorzugt von 15 bis 70 Gew.-%, stärker bevorzugt von 20 bis 60 Gew.-% und am stärksten bevorzugt von 25 bis 55 Gew.-% und
- für eine Emulsion bevorzugt im Bereich von 1% bis 98%
- für ein Gel oder Hydrogel bevorzugt 0,1 bis 99,8 Gew.-%, bevorzugt von 10 bis 99 Gew.-%, ebenfalls bevorzugt von 15 bis 80 Gew.-%, besonders bevorzugt von 20 bis 70 Gew.-%, stärker bevorzugt von 30 bis 70 Gew.-% und am stärksten bevorzugt von 35 bis 65 Gew.-%.

Der Fachmann wird insbesondere abhängig von der vorliegenden Indikation, dem Zustand des zu behandelnden Organismus (Patienten), der Schwere der Erkrankung usw. die geeignete Konzentration wählen.

In einer offenbarten Ausführungsform enthält eine erfindungsgemäß verwendbare Formulierung weiterhin mindestens ein anorganisches oder organisches Lösungsmittel. Bevorzugt ist das Lösungsmittel ausgewählt ist aus der Gruppe, bestehend aus Ethanol, Wasser und Glycerol sowie Mischungen davon.

Ein zu behandelnder Organismus im Sinne der vorliegenden Erfindung ist ein tierischer Organismus, vor allem ein Wirbeltier, insbesondere ein Säugetier, vor allem ein Mensch, Pferd, Schwein, Rind, Ziege, Schaf, Katze und Hund.

Die vorliegende Erfindung wird nachfolgend anhand von Beispielen und Figuren weiter erläutert, wobei diese die Gegenstände der Erfindung nicht beschränken.

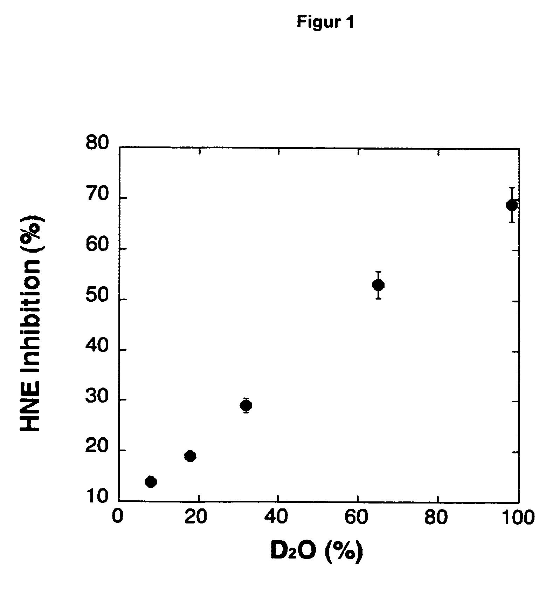

Figur 1 zeigt die Hemmung des Umsatzes des synthetischen Substrats N-Methoxysuccinyl-Ala-Ala-Pro-Val-pNitro-Anilid (AAPV) durch Humane Neutrophilen Elastase (HNE) in Abhängigkeit des Volumenanteils von D20 in der Mischung von HNE und AAPV. Die Messung des AAPV Umsatzes erfolgte spektrophotometrisch bei einer Wellenlänge von 405 nm.

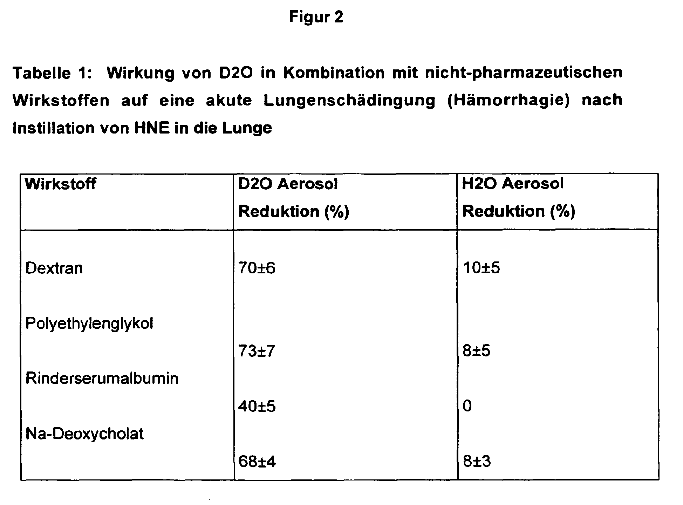

Figur 2 zeigt Tabelle 1 und die akute Lungenschädingung (Hämorrhagie) nach Instillation der Serinprotease Elastase in die Hamsterlunge, ermittelt durch spektrophotometrische Bestimmung der Hämoglobinkonzentration in der Waschlösung nach einer BAL. Dargestellt ist prozentuale Reduktion (%-Reduktion) der akuten Lungenschädigung durch D2O- und H2O-Aerosole, die mit verschiedenen weiteren nicht-pharmazeutischen Wirkstoffen versehen wurden (bezogen auf die nicht mit Aerosol behandelte Kontrollgruppe). Die Konzentration des nicht-pharmazeutischen Wirkstoffes im Aerosol betrug jeweils 1 Gew.-%. Die prozentuale Reduktion wurde aus der Verringerung der Hämoglobinkonzentration in der Waschlösung berechnet.

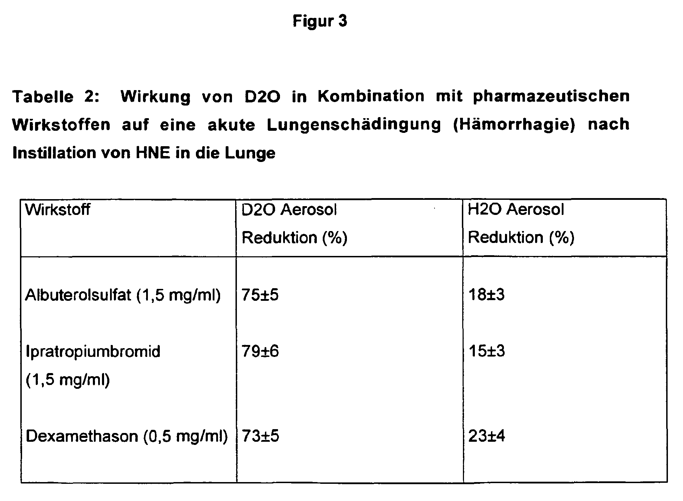

Figur 3 zeigt Tabelle 2 und die akute Lungenschädingung (Hämorrhagie) nach Instillation der Serinprotease Elastase in die Hamsterlunge, ermittelt durch spektrophotometrische Bestimmung der Hämoglobinkonzentration in der Waschlösung nach einer BAL. Dargestellt ist prozentuale Reduktion (%-Reduktion) der akuten Lungenschädigung durch D2O- und H2O-Aerosole, die mit verschiedenen weiteren pharmazeutischen Wirkstoffen versehen wurden (bezogen auf die nicht mit Aerosol behandelte Kontrollgruppe). Die Konzentration des weiteren pharmazeutischen Wirkstoffes im Aerosol ist in Klammern angegeben. Die prozentuale Reduktion wurde aus der Verringerung der Hämoglobinkonzentration in der Waschlösung berechnet.

### Beispiele

Das für alle Beispiele verwendete D20 (CU Chemie, Uetingen, Schweiz) wies eine Isotopenreinheit von 98% auf. Das verwendete H2O war destilliert und ionenausgetauscht. Sowohl D20 als auch H2O waren steril.

### Beispiel 1: Herstellung von Hydrogelen auf Basis von Acrylsäure

Es wurde 2,0 Gew.-% Carbopol 980 (Hersteller: Noveon, Inc. , 9911 Brecksville Rd., Cleveland, OH 44141-3247, USA) in getrennten Ansätzen in reinem D20, in reinem H2O oder in einer Mischung aus D20 und H2O durch Rühren gelöst und anschließend durch Pipettieren von 10 M NaOH Lösung auf einen pH Wert von 6,8 titriert. Anschließend wurden die durch die NaOH Zugabe infolge Quervernetzung der Polyacrylsäure über ihre Carboxylgruppen mit den alkalischen Hydroxyl-Gruppen entstandenen farblosen, transparenten und optisch klaren Acrylsäuregele (Carbopolgele) (D2O-Carbopolgel, H2O-Carbopollgel, D2O/H2O-Carbopollgel) bei Raumtemperatur bis zur weiteren Verwendung, für mindestens 24 Stunden, gelagert.

### Beispiel 2: Herstellung von Hydrogelen auf der Basis von Siloxanen (Silicon)

Es wurde 3,0 Gew-% Hexamethyldisiloxane (Handelsname SILMOGEN CARRIER von DOW Corning) und 1 Gew-% Ethanol in getrennten Ansätzen in reinem D20, in reinem H2O oder in einer Mischung aus D20 und H2O durch Rühren gelöst. Diese Lösungen wurden anschließend sofort im Gewichtsverhältnis 1:2 (Siliconlösung:Carbopolgel) unter kräftigen Rühren mit dem nach Beispiel 1 hergestellten Gel (Carbopolgel) gemischt, bis optisch transparente Gele (Silicongele) (D20-Silicongel, H2O-Silicongel, D2O/H2O-Silicongel) entstanden waren. Die Lagerung der Gele erfolgte bei Raumtemperatur bis zur weiteren Verwendung, für mindestens 24 Stunden.

### Beispiel 3: Herstellung von Hydrogel auf der Basis von Alginaten

Es wurde 4,0 Gew-% Alginsäurenatriumsalz (Na-Alginat) (Hersteller: Röhm GmbH Darmstadt, Deutschland) in getrennten Ansätzen in reinem D20, in reinem H2O oder in einer Mischung aus D20 und H2O durch Rühren dispergiert und anschließend durch Pipettieren von 10 M NaOH Lösung auf einen pH Wert von 7,0 titriert. Die entstandenen gelblich-braunen, transparenten Gele (Alginatgele) (D2O-Alginatgel H2O-Alginatgel, D20/H2O-Alginatgel) wurden bei Raumtemperatur bis zur weiteren Verwendung, für mindestens 24 Stunden, gelagert.

### Beispiel 4: Herstellung von Hydrogel auf der Basis von PVA

Es wurde 20 Gew-% Polyvinylalkohol (PVA C-25, Shin-Etsu Chemical Co., Japan) in getrennten Ansätzen in reinem D20, in reinem H2O oder in einer Mischung aus D2O und H2O durch Rühren gelöst. Anschließend wurden die Lösungen 5 Gefrier-Auftau-Zyklen ("freeze-thaw-cycles") unterworfen. Das Ergebnis waren Gele (PVA-Gele) (D20-PVA-Gel, H2O-PVA-Gel, D2O/H2O-PVA-Gel) mit gummiartigen Eigenschaften, welches in 2 mm dünne Scheiben geschnitten wurde. Die Gele wurden bei Raumtemperatur bis zur weiteren Verwendung, für mindestens 24 Stunden, gelagert.

### Beispiel 5: Herstellung von Hydrogel - Folien bzw. Platten auf der Basis von Agarose

Es wurde 3,0 Gew.-% Agarose in getrennten Ansätzen in reinem D20, in reinem H2O oder in einer Mischung aus D2O und H2O gelöst und die Lösungen anschließend auf 90°C erhitzt. Das verwendete D20 der Firma Sigma-Aldrich (München, Deutschland) wies eine Isotopenreinheit von 98,5% auf. Die heißen Lösungen wurde in geeignete Petrischalen auf 1,0 - 1,5 mm Höhe gegossen und abgekühlt. Die so erhaltenen Gele (Agarosegele) (D2O-Agarosegele, H2O-Agarosegele, D2O/H2O-Agarosegele) wurden unter sterilen Bedingungen bei 4 °C gelagert.

### Beispiel 6: Herstellung von Hydrogel - Folien bzw. Platten auf der Basis von Acrylamid

Es wurden Acrylamidgele (5% Acrylamid) hergestellt, wobei in getrennten Ansätzen reines D20, reines H2O oder eine Mischung aus D20 und H2O vor Zugabe des Acrylamids (mit 2,4% bis-Acrylamid) entgast und auf 40 °C erwärmt wurde. Nach Zugabe von Acrylamid und bis-Acrylamid wurden die Lösungen gemischt (Vortex Mischer, 1 min. bei 200 U/min) und anschließend die Katalysatoren Tetramethyl-ethylendiamin (TEMES ; 1,0%) und Ammoniumpersulfat (AP ; 0,1%) zugegeben, gefolgt von 10 Sek. Mischen. Dann wurden die Gele in Petrischalen gegossen (Höhe des Gels 1,0-1,5 mm) und für 2 Stunden bei 40° C gelagert. Anschließend wurden die Gele (D20-Acrylamidgel, H2O-Acrylamidgel, D2O/H2O-Acrylamidgel) gewaschen, wobei zum Waschen das analoge Wassergemisch wie zum Hydratisieren des Gels (reines D20, reines H2O oder eine Mischung aus D20 und H2O) verwendet wurde. Die Gele wurden bei Raumtemperatur bis zur weiteren Verwendung, für mindestens 24 Stunden, gelagert.

### Beispiel 7: Herstellung einer D20-haltigen Creme

Zu 50 Gramm Asche Basiscreme (Hersteller: Asche Chiesi GmbH, Hamburg, Deutschland) wurde bei 40 °C unter ständigen Rühren langsam D20 hinzugegeben, bis ein Gewichtsanteil vom 38% D20 (bezogen auf das Ausgangsgewicht der Creme) in der homogenen Mischung erreicht war. Die Creme wurde anschließend auf Raumtemperatur abgekühlt und luftdicht verschlossen gelagert.

### Beispiel 8: Erzeugung von D20-Aerosolen

Das für alle Beispiele verwendete D20 (CU Chemie, Uetingen, Schweiz) wies eine Isotopenreinheit von 98% auf. Das verwendete H2O war destilliert und ionenausgetauscht. Sowohl D20 als auch H2O waren steril. Zur Aerosolisierung verwendet wurde ein Pari LC Plus Universal Vernebler (PARI GmbH, Starnberg, Deutschland) in Kombination mit einem Pari Universal Kompressor, der 200 mg/Min polydisperses Aerosol mit einer mittleren Partikelgröße (medianer Massendurchmesser) von 2,5 µm für reines H2O und reines D20 und von 2.5-4,5 µm für H2O bzw. D2O mit zusätzlichen nicht-pharmazeutischen und/oder pharmazeutischen Wirkstoffen erzeugte (Betriebsdruck 2,0 bar, Durchflussmenge der Kompressorluft war 6,0 I/Min.) Die Partikelgrößenmessung erfolgte mittels dynamischer Lichtstreuung in einer Durchfluss-Küvette Die Aerosolerzeugung erfolgte bei einer Temperatur von 37°C durch entsprechende Thermostatierung des Verneblers in einem Wasserbadthermostaten.

### Beispiel 9: Hemmung des Enzyms Humane Neutrophilen Elastase (HNE)

HNE wurde zusammen mit dem synthetischen Substrat N-Methoxysuccinyl-Ala-Ala-Pro-Val-pNitro-Anilid (AAPV) inkubiert. Der Umsatz von AAPV wurde dabei photometrisch bei 405 nm bestimmt. Folgendes Inkubationsschema kam zur Anwendung:
Probe 1) HNE in D20 mit AAPV in D20;
Probe 2) HNE in D20 mit AAPV in H2O;
Probe 3) HNE in H2O mit AAPV in D20;
Kontrolle: HNE in H2O mit AAPV in H2O.

Hierbei wurden Enzym und Substrat jeweils für 60 Min. mit H2O bzw. D2O inkubiert bevor HNE und AAPV zusammen gegeben wurden. Die Menge an D20 wurde bei den Versuchen über einen Bereich von 10 bis 100 % variiert, um die Abhängigkeit der Inhibition des AAPV Umsatzes durch HNE nachzuweisen. Abbildung 1 zeigt die Inhibition des AAPV Umsatzes als Funktion der D20 Menge (vol%) in der Mischung. Daraus wurde eine halb-maximale effektive Dosis bei einem D20 Gehalt von ca. 40 vol% bestimmt. Bei 100% D20 in der Mischung wurde eine Inhibition des AAPV Umsatzes von ca. 70% erzielt.

### Beispiel 10: Wirksamkeit von D20 Instillation in einem Tiermodell der COPD

Zur Untersuchung der Wirkung von D20 in einem Modell zur chronisch obstruktiven Lungenerkrankung (COPD), wurden männliche syrische Goldhamster (mittleres Gewicht 118g ± 4 g) in zwei Gruppen (Versuchsgruppe und Kontrollgruppe) zu je 10 Hamstern aufgeteilt. Die Tiere beider Gruppen wurden zunächst intraperitoneal anästhesiert (Ketamin 95 mg/kg, und Xylazin 17 mg/kg) und anschließend mittels direkter Laryngoskopie endotracheal intubiert. Die Versuchsgruppe erhielt 250 µl einer 0,9% NaCl-Lösung auf D20 Basis (98,5% Deuteriumanreicherung) instilliert, während bei der Kontrollgruppe 250 µl einer entsprechenden isotonischen Kochsalzlösung auf H2O Basis verwendet wurde. Jedes Tier erhielt nach Abschluss dieser Instillation in einem zeitlichen Abstand von 30 Minuten eine zweite Instillation, welche für beide Gruppen identisch war: 100 µl humane neutrophile Elastase (HNE, 500 µg/ml in steriler 0.9% NaCl-Lösung). Die Tiere wurden 4 Stunden (6 Tiere pro Gruppe) bzw. 24 Stunden (4 Tiere pro Gruppe) nach der letzten Instillation getötet (Phenobarbital 70 mg/kg intraperitoneal).

Für die 4 Stunden nach der Instillation getöteten Tiere wurde eine BAL (broncho-alveoläre Lavage) durchgeführt. Für die BAL wurde 2,5 ml 0,9% NaCl-Lösung wiederholt (insgesamt 3mal) instilliert und anschließend die Hämoglobinkonzentration im Instillat spektrophotometrisch bestimmt. Der so erhaltene Wert wurde als Maß für die Schädigung der Lunge (Hämorrhagie) durch die humane neutrophile Elastase (HNE) verwendet. Für die Kontrollgruppe wurde ein mittlerer Hämoglobinwert von 320±50 mOD (Milli-Optische Dichte) erhalten. Für die Versuchsgruppe wurde ein mittlerer Hämoglobinwert von 170±20 mOD gemessen. Diese Reduktion der Hämoglobinkonzentration in der BAL entspricht einer Inhibition der durch Elastase verursachten akuten Lungenschädigung (Hämorrhagie) von 47%.

Für die 24 Stunden nach der Instillation getöteten Tiere wurde die Lunge entnommen, in Formalin fixiert und in Paraffin eingebettet. Histologische Längschnitte der Lunge in Richtung Lungenhilus wurden angefertigt, gefärbt und mittels einer histochemischen Reaktion (Berliner Blau-Reaktion) wurde Eisen sichtbar gemacht, dass als Mass für die Stärke der HNE-induzierten Hämorrhagie diente. Die mikroskopische Untersuchung der Schnitte erbrachte folgenden Befund:
Kontrollgruppe: Hohe Anzahl blaugefärbter Zellen (Makrophagen, welche Blutreste internalisiert hatten) als Ausdruck einer ausgeprägten Hämorrhagie.
Versuchsgruppe: Signifikant geringere Anzahl blaugefärter Zellen als Ausdruck einer geringeren Hämorrhagie

### Beispiel 11: Wirksamkeit von D20 Aeorsolen bei einem Tiermodell der COPD

Männliche syrische Goldhamster (mittleres Gewicht 105g ± 8 g) wurden in zwei Gruppen (Versuchsgruppe und Kontrollgruppe) zu je 6 Hamstern aufgeteilt. Die Tiere beider Gruppen wurden zunächst intraperitoneal anästhesiert (Ketamin 95 mg/kg, and Xylazin 17 mg/kg) und anschließend mittels direkter Laryngoskopie endotracheal intubiert. Beide Gruppen erhielten 100 µl humane neutrophile Elastase (500 µg/ml in steriler 0.9% NaCl-Lösung) instilliert. Anschließend wurden die Tiere in getrennte Käfige verbracht und einer kontinuierlichen Behandlung mit D20-Aerosol (Versuchsgruppe) oder H2O-Aerosol (Kontrollgruppe) (hergestellt nach Beispiel 8) in der Atemluft (Luftfeuchtigkeit 60%) für 24 Stunden behandelt. Nach dieser Behandlung wurden die Tiere getötet (Phenobarbital 70 mg/kg intraperitoneal) und eine BAL (bronchoalveoläre Lavage) durchgeführt. Für die BAL wurde 2,5 ml 0,9% NaCl-Lösung wiederholt (insgesamt 3mal) instilliert und anschließend die Hämoglobinkonzentration im Instillat spektrophotometrisch bestimmt. Der so erhaltene Wert wurde als Maß für die Schädigung der Lunge (Hämorrhagie) durch die humane neutrophile Elastase (HNE) benutzt. Für die Kontrollgruppe wurde ein mittlerer Hämoglobinwert von 300±50 mOD (Milli-Optische Dichte) erhalten. Für die Versuchsgruppe wurde ein mittlerer Hämoglobinwert von 120±20 mOD gemessen. Die Reduktion der Hämoglobinkonzentration in der Versuchsgruppe entspricht einer Inhibition der durch Elastase verursachten akuten Lungenschädigung (Hämorrhagie) von 60%.

### Beispiel 12: Nachweis der Wirksamkeit von D20 Aerosolen, die mit nicht-pharmazeutischen Wirkstoffen versetzt wurden, an einem Tiermodell der COPD

Männliche syrische Goldhamster (mittleres Gewicht 105g ± 8 g) wurden in zwei Gruppen (D20-Gruppe und H2O-Gruppe) zu je 8 Hamstern aufgeteilt. Die beiden Gruppen wurden anschließend in Untergruppen von je 2 Hamstern aufgeteilt, also 4 D20-Gruppen und 4 H2O-Gruppen. Zusätzlich wurde eine dritte Gruppe (Kontrollgruppe) aus 2 Hamstern geschaffen. Die Tiere aller drei Gruppen wurden zunächst intraperitoneal anästhesiert (Ketamin 95 mg/kg, and Xylazin 17 mg/kg) und anschließend mittels direkter Laryngoskopie endotracheal intubiert. Beide Gruppen erhielten 100 µl humane neutrophile Elastase (500 µg/ml in steriler 0.9% NaCl-Lösung mit H2O) instilliert. Anschließend wurden die Tiere der D20- und H2O-Gruppen in getrennte Käfige (je 2 Hamster pro Käfig) verbracht und einer kontinuierlichen Behandlung mit D20-Aerosol (D20-Gruppe) und H2O-Aerosol (H2O-Gruppe) (hergestellt nach Beispiel 8) in der Atemluft (Luftfeuchtigkeit 60%) für 4 Stunden unterzogen. Die D20- bzw. H2O-Aerosole enthielten in der zu ihrer Erzeugung nach Beispiel 8 verwendeten Ausgangslösung (d.h. die Lösung vor der Aerosolisierung) zusätzlich jeweils eine der folgenden Substanzen mit den in Klammern angegebenen Konzentrationen: Dextran 4000 (1,0 Gew.-%), Polyethylenglykol 4000 (1,0 Gew.-%), Serumalbumin vom Rind (1,0 Gew.-%), Natrium-Deoxycholat (1,0 Gew.-%). Die Tiere wurden mit den entsprechenden Aerosolen (D20 bzw H2O) für 4 Stunden behandelt, die relative Luftfeuchtigkeit im Käfig betrug hierbei 60%. Nach dieser Behandlung wurden alle Tiere (einschl. der 2 Tiere der nicht mit Aerosol behandelten Kontrollgruppe) getötet (Phenobarbital 70 mg/kg intraperitoneal) und eine BAL (bronchoalveoläre Lavage) durchgeführt. Für die BAL wurde 2,5 ml 0,9% NaCl-Lösung wiederholt (insgesamt 3mal) instilliert und anschließend die Hämoglobinkonzentration im Instillat spektrophotometrisch bestimmt. Der so erhaltene Wert wurde als Maß für die Schädigung der Lunge (Hämorrhagie) durch die humane neutrophile Elastase (HNE) benutzt. **Tabelle 1** gibt die aus der jeweiligen Hämoglobinkonzentration berechnete prozentuale Reduktion der durch HNE verursachten akuten Lungenschädigung.

### Beispiel 13: Nachweis der Wirksamkeit von D20 Aerosolen, die mit weiteren pharmazeutischen Wirkstoffen versetzt wurden, an einem Tiermodell der COPD

Als zusätzliche pharmazeutische Wirkstoffe wurden aus der Gruppe der Bronchodilatatoren wurden der Beta-Agonist Albuterol (Albuterolsulfat) und das Anticholinergika Ipratropium (Ipratropiumbromid) sowie aus der Gruppe der Steroide das Glucocortikoid Dexamethason ausgewählt und in Kombination mit D2O-Aerosolen in Bezug auf ihre Wirksamkeit zur Behandlung von COPD geprüft. Männliche syrische Goldhamster (mittleres Gewicht 105g ± 8 g) wurden in zwei Gruppen (D2O-Gruppe und H2O-Gruppe) zu je 6 Hamstern aufgeteilt. Die beiden Gruppen wurden anschließend in drei Untergruppen von je 2 Hamstern aufgeteilt, also 3 D2O-Gruppen und 3 H2O-Gruppen. Zusätzlich wurde eine dritte Gruppe (Kontrollgruppe) aus 2 Hamstern geschaffen. Die Tiere aller drei Gruppen wurden zunächst intraperitoneal anästhesiert (Ketamin 95 mg/kg, and Xylazin 17 mg/kg) und anschließend mittels direkter Laryngoskopie endotracheal intubiert. Beide Gruppen erhielten 100 µl humane neutrophile Elastase (500 µg/ml in steriler 0.9% NaCl-Lösung) instilliert. Anschließend wurden die Tiere der D20- und H2O-Gruppen in getrennte Käfige (je 2 Hamster pro Käfig) verbracht und einer kontinuierlichen Behandlung mit D2O-Aerosol (D2O-Gruppe) und H2O-Aerosol (H2O-Gruppe) (hergestellt nach Beispiel 8) in der Atemluft (Luftfeuchtigkeit 60%) für 4 Stunden unterzogen. Die D2O- bzw. H2O-Aerosole enthielten in der zu ihrer Erzeugung nach Beispiel 8 verwendeten Ausgangslösung (d.h. die Lösung vor der Aerosolisierung) zusätzlich jeweils eine der folgenden Substanzen mit den in Klammern angegebenen Konzentrationen: Albuterolsulfat (1,5 mg/ml), Ipratropiumbromid (1,5 mg/ml), Dexamethason (0,5 mg/ml). Die Tiere wurden mit den entsprechenden Aerosolen (D2O bzw. H2O) in der Atemluft für 4 Stunden behandelt, die relative Luftfeuchtigkeit im Käfig betrug hierbei 60%. Nach dieser Behandlung wurden alle Tiere (einschl. der 2 Tiere der nicht mit Aerosol behandelten Kontrollgruppe) getötet (Phenobarbital 70 mg/kg intraperitoneal) und eine BAL (bronchoalveoläre Lavage) durchgeführt. Für die BAL wurde 2,5 ml 0,9% NaCl-Lösung wiederholt (insgesamt 3mal) instilliert und anschließend die Hämoglobinkonzentration im Instillat spektrophotometrisch bestimmt. Der so erhaltene Wert wurde als Maß für die Schädigung der Lunge (Hämorrhagie) durch die Serinprotease (Elastase) benutzt. Tabelle 2 zeigt die aus der jeweiligen Hämoglobinkonzentration berechnete prozentuale Reduktion der durch HNE verursachten akuten Lungenschädigung.

### Referenz-Beispiel 14: Nachweis der Wirksamkeit von D20 zur Hemmung der Inflammation im Myokardgewebe

In einem Tiermodell zum Reperfusionsschaden wurde bei männlichen, weißen Neuseeland-Kaninchen durch Ligatur einer Koronararterie eine Minderdurchblutung des Herzmuskelgewebes erreicht, was zur Schädigung führte (Bidouard JP et al.; SSR69071, an elastase inhibitor, reduces myocardial infarct size following ischemia-reperfusion injury. Eur J Pharmacol. 2003;461:49-52). Durch Thorakotomie wurde ein Seitenast der linken Koronararterie durch Ligatur für 30 Minuten verschlossen und anschließend wieder eröffnet um eine Reperfusion über 120 Minuten zu erreichen. Vor Entnahme des Herzens wurde ein Tintenbolus (Pelikantusche) in die aszendente Aorta gegeben. Zur Untersuchung der Wirkung von D20 auf den Reperfusionsschaden wurden die Tiere in drei Gruppen zu je 2 Tieren eingeteilt. Zwei Gruppen erhielten entweder 15 Minuten vor (Versuchsgruppe 1) oder 25 Minuten nach (Versuchsgruppe 2) der Wiedereröffnung der Koronararterie eine intravenöse Behandlung mit D20 in Form einer isotonen Kochsalzlösung, die andere Gruppe (Kontrollgruppe) wurde mit H2O als isotone Kochsalzlösung behandelt. Nach Entnahme des Herzens, Abklemmen der Aorta, Formalinfixierung und Paraffineinbettung wurde das Herz auf die Homogenität der Tintenverteilung im Kapillarsystem durch 3 Querschnitte sowie immunhistochemisch untersucht. Der immunhistochemische Nachweis von Granulozyten erfolgte durch alpha Naphthol Chloracetat Esterase-Färbung. Bei den Tieren der Kontrollgruppe wurde ein signifikant höherer Gehalt an Granulozyten und deutlich geringere Kapillarmarkierung durch Pelikantusche festgestellt. Histologische Unterschiede zwischen den Versuchsgruppen 1 und 2 waren nicht signifikant. Es konnte somit nachgewiesen werden, dass in den D20-behandelten Gruppen ein signifikant geringerer Herzmuskelschaden als bei den H2O-behandelten Tieren auftrat.

### Referenz-Beispiel 15: Nachweis der Wirksamkeit von D20 Hemmung der Inflammation im Hirn nach einer cerebralen Ischämie

In einem Tiermodell wurde die Wirkung von D20 auf die cerebrale Ischämie untersucht. Hierzu wurde bei erwachsenen Long-Evans Ratten durch Verschluss beider A. carotis für eine Stunde eine cerebrale Ischämie erzeugt, der eine Reperfusion über 24 Stunden folgte. Es erfolgte eine Nachbeobachtung mit Evaluierung neuro-vegetativer Symptome (Spontanaktivität, koordiniertes Gehen, Vorfußstrecken und Klettern). Die Schädigung des Hirngewebes wurde anschließend histologisch untersucht. In zwei Versuchsgruppen zu je 3 Ratten wurde den Tieren 15 Minuten vor Einleitung der Ischämie und unmittelbar vor der folgenden Reperfusion entweder D20- (Versuchsgruppe) oder H2O-(Kontrollgruppe) haltige isotone Kochsalzlösung infundiert.

Hierbei zeigte sich ein signifikanter Effekt auf die Verminderung des Reperfusionsschadens (Histologischer Nachweis analog dem Beispiel 10) nur in den mit D20 behandelten Gruppen sowohl in Bezug auf das Vorhandensein von Granulozyten in histologischen Schnitten als auch bezüglich des neurologischen Verhaltens der Tiere nach der Operation.

### Referenz-Beispiel 16: Wirksamkeit von D20 bei bullösem Pemphigoid

In einem ex vivo-Modell wurde die Blasenflüssigkeit bei Patienten mit bullösem Pemphigoid entnommen (Verraes et al., 2001). Anschließend wurde rekombinantes, radioaktiv markiertes BP180-Antigen, das Zielantigen bei dieser Erkrankung, der Blasenflüssigkeit zugesetzt und der Abbau autoradiographisch nach Gelelektrophorese bestimmt. Zur Hemmung der Elastase-Aktivität in der Blasenflüssigkeit wurden dieser entweder D2O oder der Elastase-Inhibitor Chloromethylketon zugesetzt. Als Vergleich diente eine unbehandelte Kontrolle. Sowohl mit Chloromethylketon als auch mit D2O ließ sich der Abbau von rekombinantem BP180-Antigen hemmen, während das Protein von der unbehandelten Blasenflüssigkeit nahezu vollständig degradiert wurde.

### Referenz-Beispiel 17: Wirksamkeit von D2O bei Psoriasis vulgaris

Zur Bestimmung der Wirkung von D2O bei Psoriasis vulgaris wurde ein Halbseitenversuch mittels eines Hydrogels durchgeführt. Bei dem Gel handelte es sich um 2 Gew.-% Carbopol 980 mit 1 Gew.-% Harnstoff. Der pH Wert des Gels wurde mittels NaOH Lösung auf 6,5 eingestellt. Es wurden Patienten behandelt, die vergleichbare Läsionen an beiden Ellenbogen aufwiesen. Zur Evaluierung wurde der "local psoriasis severity index" (LPSI) verwendet (Henneicke-von Zepelin HH, Mrowietz U, Färber L, Bruck-Borchers K, Schober C, Huber J, Lutz G, Kohnen R, Christophers E, Welzel D. Highly purified omega-3-polyunsaturated fatty acids for topical treatment of psoriasis. Results of a double-blind, placebocontrolled multicentre study. Br J Dermatol. 1993;129:713-7). Die Patienten wurden angewiesen, den Inhalt der mit "rechts" und "links" markierten Tuben 2x/Tag auf die entsprechenden Läsionen auf dem rechten bzw. linken Ellenbogen aufzutragen. In der einen Tube befand sich ein Gel, dass mit D2O hergestellt wurde, in der anderen wurde das Gel mit H2O angefertigt. Die Zuteilung erfolgte zufällig. Der LPSI wurde einmal wöchentlich bestimmt. Nach 4 Wochen Therapie und Zuordnung der Applikationen zu den jeweiligen Seiten zeigte sich eine signifikante Abnahme des LPSI bei den D2O-behandelten Läsionen im Vergleich zu denen, auf die das H2O-Gel aufgetragen wurde.

### Referenz-Beispiel 18: Wirksamkeit von D2O bei Peritonitis und septischen Schock

Die Wirkung von D2O zur Anwendung bei einer Peritonealdialyse wurde in einem Tiermodell zur infektiösen Peritonitis untersucht (Welten AG, Zareie M, van den Born J, ter Wee PM, Schalkwijk CG, Driesprong BA, Mul FP, Hordijk PL, Beelen RH, Hekking LH. In vitro and in vivo models for peritonitis demonstrate unchanged neutrophil migration after exposure to dialysis fluids. Nephrol Dial Transplant. 2004;19:831-9.). Dabei wurde männlichen Wistar-Ratten ein Katheder zur Peritonealdialyse gelegt und zur Induktion einer Peritonitis sowie zur weiteren Peritonealdialyse verwendet. Eine eitrige Peritonitis wurde durch Einspülung einer Suspension von S. aureus ATCC 25923 (1 x 10⁹ c.f.u in 0.5 ml) erzeugt. Nach 4 Stunden wurde eine peritoneale Lavage entweder mit isotoner Kochsalzlösung oder mit einer isotonen Kochsalzlösung mit 30%/H2O 70% durchgeführt. Nach dem Tod der Tiere spätestens aber nach 24 Stunden wurden die bakterielle Besiedlung sowie die entzündliche Reaktion im Peritoneum histologisch untersucht. Dabei zeigte sich sowohl eine verminderte Anzahl von Bakterien in der quantitativen Kultur als auch eine geringere Entzündungsaktivität bei den mit D2O behandelten Tieren.

### Referenz-Beispiel 19: Wirksamkeit von D20 bei allergischen Erkrankungen

In einem Versuch wurde ein Extrakt der Hausstaubmilbe Dermatophagoides pteronissinus (D. pter) lyophilisiert und in D2O aufgenommen. Anschließend wurde der D. pter/D2O-Extrakt in einem Pric-Test bei Patienten untersucht, die auf die konventionelle Testflüssigkeit mit D. pter positiv reagiert hatten. Zur Kontrolle dienten erneut die unbehandelte Testlösung, Histamin-Lösung 0,1%, die mit H2O angesetzt wurde, Histamin-Lösung 0,1%, die mit D2O angesetzt wurde, sowie isotone Kochsalzlösung. Es zeigte sich, dass unter Verwendung der D2O/D. pter-Testlösung nur eine sehr geringe Quaddelreaktion auslösbar war, wohingegen die Quaddelreaktion mit der unbehandelten D. pter-Lösung etwa der der Histamin-Kontrolle entsprach. Es zeigten sich keine Unterschiede zwischen den D2O- und H2O-Histamin pric Tests.

Dementsprechend wurde eine Lösung mit D2O zur Anwendung als Nasenspray hergestellt. Jeweils 5 Patienten mit nachgewiesener Typ-1 Sensibilisierung und allergischer Rhinitis wurden entweder mit dem D2O-haltigen Nasenspray (isotone Kochsalzlösung unter Verwendung von D2O 70%) oder mit isotoner Kochsalzlösung mit H2O behandelt. Als Parameter wurden die Zahl von Niesattacken bestimmt und Intensität von Fließschnupfen sowie nasaler Juckreiz mittels visueller Analogskalen gemessen. Hierfür wurde ein Patiententagebuch verwendet. Nach einer Versuchsdauer von 14 Tagen und Gebrauch der jeweiligen Sprays 6x täglich zeigte sich in der mit D2O-behandelten Gruppe eine signifikante Abnahme der erhobenen Parameter im Vergleich zur H2O-Gruppe.

### Referenz-Beispiel 20: Wirksamkeit von D20 zum Abschwellen der Nasenschleimhaut

Es wurde physiologische Kochsalzlösung aus D2O (Versuchsgruppe) und H2O (Kontrollgruppe) hergestellt und in Pumpspraybehälter gefüllt, wie sie üblicherweise zur Freisetzung von Nasenspray genutzt werden. Probanden mit Erkältungskrankheiten, die von starken Nasenverstopfungen begleitet waren, wurden in 2 Gruppen zu je 8 Probanden eingeteilt. Der Luftdurchfluss durch die Nase wurde bei jedem Probanden unmittelbar vor der Behandlung (nach gründlicher Entfernung von Sekreten) mittels Rhinomanometrie bestimmt (Anfangswert). Anschließend erhielten die Probanden der Versuchsgruppe je einen Pumpstoß D2O Saline in jedes Nasenloch während die Probanden der Kontrollgruppe stattdessen H2O Saline in gleicher Menge erhielten. Jeweils 30 und 60 Minuten nach der Applikation wurde der Luftdurchfluss bei jedem Probanden mittels Rhinomanometrie bestimmt und die prozentuale Veränderung des Luftdurchflusses zum Anfangswert berechnet. Aus diesen Werten wurde für Verum- und Kontrollgruppe ein Mittelwert gebildet (mittlere prozentuale Veränderung des Luftdurchflusses, <L_{f-}-%>). Ein positiver Wert von <L_{f-}-%> bedeutet hierbei eine Eröhung, ein negativer Wert eine Erniedrigung des Luftdurchflusses. Für die Versuchsgruppe ergab sich nach 30 Min. eine Wert von <L_{f-}-%> = 20±5 % und nach 60 Min. <L_{f-}-%> = 90±10 %. Die Kontrollgruppe ergab nach 30 Min. <L_{f-}-%> = 10±5% und nach 60 Min. <L_{f-}-%> = 5±5%. Es konnte somit eine signifikante Erhöhung des Luftdurchflusses und eine damit verbundene Abschwellung der Nasenschleimhäute für die Versuchsgruppe (D2O) gegenüber der Kontrollgruppe (H2O) nachgewiesen werden.

### Referenz-Beispiel 21: Wirksamkeit von D20 zur Therapie von akuter Nasen-Nebenhöhlenentzündung (Sinusitis)

Aerosole aus D2O und H2O, jeweils versetzt mit 150 mM NaCL, wurden nach Beispiel 2 bereitgestellt. Probanden mit einer akuten Sinusitis im Kieferhöhlenbereich wurden in zwei Gruppen eingeteilt. Die Versuchsgruppe inhalierte für 15 Minuten das D2O Aerosol, die Kontrollgruppe das H2O Aerosol. Beim Inhalieren wurden die Probanden angewiesen, möglichst weitgehend durch die Nase einzuatmen. Jeweils 3 Stunden nach der Inhalation wurde eine rhinoendoskopische Untersuchung der Nebenhöhlen durchgeführt. Es wurde eine signifikante Reduktion der Schleimhautschwellung der Versuchsgruppe gegenüber der Kontrollgruppe festgestellt. Dies führte bei der Versuchsgruppe zu einem verbesserten Abfluss von Sekreten.

### Referenz-Beispiel 22: Wirksamkeit von D20 bei allergischer Rhinitis

Dem Beispiel 21 entsprechend wurde eine Lösung mit D2O zur Anwendung als Nasenspray hergestellt. Jeweils 5 Patienten mit nachgewiesener Typ-1 Sensibilisierung und allergischer Rhinitis wurden entweder mit dem D2O-haltigen Nasenspray (isotone Kochsalzlösung unter Verwendung von D2O 70%) oder mit isotoner Kochsalzlösung mit H2O behandelt. Als Parameter wurden die Zahl von Niesattacken bestimmt und Intensität von Fließschnupfen sowie nasaler Juckreiz mittels visueller Analogskalen gemessen. Hierfür wurde ein Patiententagebuch verwendet. Nach einer Versuchsdauer von 14 Tagen und Gebrauch der jeweiligen Sprays 6x täglich zeigte sich in der mit D2O-behandelten Gruppe eine signifikante Abnahme der erhobenen Parameter im Vergleich zur H2O-Gruppe.

### Referenz-Beispiel 23: Wirksamkeit von D20 bei allergischer Konjunktivitis

Zur Prüfung der Wirksamkeit von Augentropfen, die mit D2O hergestellt wurden, kam ein entsprechendes Tiermodell zur Anwendung (Minami et al., Biol. Pharm. Bull 28:473-476, 2005). Bei 6 Wochen alten Wistar-Ratten wurde durch Injektion von Hühnereiweiß zusammen mit Alaun und 10¹⁰ B. pertussis in die Fußsohlen und anschließender Boosterung mit Hühnereiweiß im Rückenbereich eine Allergie gegenüber Hühnereiweiß erzeugt. Vor dem Experiment wurden die Tiere in einem klimatisierten Käfig gehalten und nach 10minütiger Anpassung 5 µl Hühnereiweiß-Lösung in jedes Auge geträufelt. Die Tiere wurden dann in einen Beobachtungskäfig verbracht und die Kratzbewegungen in Richtung der Augen über die Zeit von 20 Minuten gezählt. Dann wurden Hyperämie und konjunktivales Ödem bestimmt.

Zur Untersuchung einer Wirkung von D2O auf die durch Hühnereiweiß hervorgerufenen Symptome wurden in einem doppel-blinden Design entweder H2O- oder D2O-basierte isotonische Kochsalzlösung zusammen mit dem Hühnereiweiß eingeträufelt. Nach 20 Minuten zeigten sich in der D2O-behandelten Gruppe im Vergleich zu den H2O-behandelten Tieren eine signifikante Abnahme der Zahl der Kratzbewegungen in Richtung Auge. Auch Hyperämie und Ödem waren in der D2O-Gruppe im Vergleich zur H2O-Gruppe reduziert. Es konnte aus diesen Versuchen der Schluss gezogen werden, dass D2O-haltige Augentropfen die Symptome einer allergischen Konjunktivitis deutlich zu reduzieren vermögen.

### Referenz-Beispiel 24: Wirksamkeit von D20 bei allergischem Asthma bronchiale

Die Wirkung von D2O beim allergischen Asthma wurde in einem Tiermodell bei 10-Wochen alten braunen Norwegischen Ratten dargestellt (Roumestan et al., Resp Res 8:35-46, 2007). Die Sensibilisierung der Tiere erfolgte durch wiederholte intraperitoneale Injektionen von Ovalbumin. Anschließend wurden die Tiere in einen klimatisierten Käfig verbracht und konstant mit einem Aerosol aus isotoner H2O- oder D2O-Lösung für 6 Tage behandelt. Anschließend erfolgte eine Asthma-Auslösung durch ein Ovalbumin-haltiges Aerosol. Als Parameter wurde die Veränderung des Atemwegswiderstandes durch barometrische Plethysmographie an den lebenden Tieren nach 24 Stunden bestimmt. Hierbei zeigte sich eine signifikante Verstärkung des Atemwegswiderstandes in der H2O-behandelten Gruppe. Bei den Tieren, die mit dem D2O-Aerosol behandelt wurden, kam es nur zu einer leichten Erhöhung des Atemwegswiderstandes. Diese Daten legen den Schluss nahe, dass D2O zur Therapie des allergischen Asthma bronchiale Anwendung finden kann.

### Referenz-Beispiel 25: Wirksamkeit von D20-haltigem Gel nach topischer Applikation auf die Progredienz der leukozytoklastischen Hautvaskulitis

Die leukozytoklastische Hautvaskulitis ist durch ein rezidivierendes Verhalten gekennzeichnet. Ein besonderes Merkmal dieser Dermatose ist die Zerstörung kutaner Kapillaren durch einen Entzündungsprozess, an dem nahezu ausschließlich neutrophile Granulozyten beteiligt sind. Zur Überprüfung der Wirkung von D2O-haltigen Gel auf das Ausmaß der kutanen Läsionen wurden Patienten mit leukozytoklastischer Hautvaskulitis in einem Halbseitenversuch behandelt. Randomisiert wurde entweder ein H2O- oder ein D2O-haltiges Gel jeweils auf den rechten, bzw. linken Unterschenkel 3x täglich aufgetragen. Nach einer Therapiedauer von 4 Tagen wurde anhand eines klinischen Scores, der die Zahl und Ausprägung vaskulitischer Läsionen beurteilt, die Therapie verglichen. Dabei zeigte sich an den Unterschenkeln, die mit D2O-Gel behandelt worden waren, eine deutliche Verminderung des klinischen Scores.

### Beispiel 26: Wirksamkeit von D2O-Infusionen bei systemischen Vaskulitiden am Beispiel der Wegenerschen Granulomatose.

In der Pathogenese der Wegenerschen Granulomatose kommt einer Aktivierung von neutrophilen Granulozyten eine wichtige Bedeutung bei. Die Krankheitsaktivtät kann durch Bestimmung von Proteinase 3-anti-neutrophilen cytoplasmatischen Antikörpern (PR3-ANCA) bestimmt werden.
Bei Patienten mit M. Wegener wurde in einer offenen Studie die Wirkung einer zusätzlichen Infusion von 500 ml einer mit 70%igem D2O hergestellten isotonen Kochsalz-Lösung einmal täglich über eine Woche zusätzlich zur individuellen systemischen Therapie bei 5 Patienten untersucht. Dabei diente die Bestimmung des PR3-ANCA im Serum vor und nach einer Woche nach der letzten D2O-Infusion als Kontrollparameter. Fünf Patienten, bei denen ausschließlich die individuelle Therapie ohne weitere Infusionen angewendet wurde, dienten als Vergleich. Dabei zeigte sich bei den Patienten, die zusätzlich zur individuellen Therapie D2O-Infusionen bekamen eine stärkere Reduktion der PR3-ANCA Werte im Vergleich zu den konventionell behandelten Patienten. Dies legt den Schluss zu, dass die systemische Anwendung von D2O zur adjunktiven Therapie von systemischen Vaskulitiden geeignet ist.

### Referenz-Beispiel 27: Wirksamkeit von D20 zur Therapie von rezidivierender benigner Aphthosis (RBA)

8 freiwillige Probanden mit der Minorform der RBA (Durchmesser der runden bis ovalen Erosionen 3-5 mm) an für eine optische Inspektion gut zugänglichen Stellen der Mundschleimhaut (insbes. Backenschleimhaut und Schleimhaut im Unterkieferbereich) wurden in 2 Gruppen zu je 4 Probanden (Versuchsgruppe und Kontrollgruppe) randomisiert eingeteilt und die Größe (Durchmesser) der Aphthen (Anfangswert) wurde optisch auf 0.5 mm genau bestimmt. Für die Größenbestimmung wurde nur die grau belegte, erodierte Fläche betrachtet, der rote Randbereich wurde nicht berücksichtigt. Die ersten Aphthosis Symptome der Probanden beider Gruppen waren mindestens 2 Tage und höchstens 4 Tage alt. Die Versuchsgruppe erhielt 3 mal täglich ein nach Beispiel 1 hergestelltes D2O Gel mittels eines geeigneten Applikators auf die Erosion (Aphthe) aufgetragen. Es wurde sichergestellt, daß das applizierte Gel die gesamte Fläche der Aphthe zuzüglich eines 2-3 mm breiten Randes bedeckte und mindestens 5 Minuten Zeit zum Einwirken hatte. Die Kontrollgruppe wurde analog mit einer identischen Dosierung und Applikation eines nach Beispiel 1 hergestellten H2O Gels behandelt. 3 Tage nach der ersten Gel Applikation (d.h. nach insgesamt 9 Applikationen pro Proband) wurde bei beiden Gruppen eine erneute Bestimmung der Aphthendurchmesser vorgenommen. Für die Versuchsgruppe konnte eine mittlere Reduzierung der Durchmesser in Bezug auf den Anfangswert von 30±10% nachgewiesen werden, bei der Kontrollgruppe wurde eine mittlere Erhöhung der Aphthendurchmesser um 15±10% beobachtet.

## Patentansprüche

1. Deuteriumoxid zur Verwendung für die Prophylaxe und/oder Therapie von Elastaseassozierten Lungenerkrankungen, ausgewählt aus aus der Gruppe, bestehend aus chronische obstruktive Lungenerkrankung (COPD), zystische Fibrose, chronische Bronchitis, Lungenfibrose, akutes Atemwegssyndrom, Lungenemphysem und Hämorrhagie, wobei die Aktivität der Elastase gehemmt oder inhibiert wird.

2. Deuterium zur Verwendung nach Anspruch 1, wobei es sich bei der Elastase um die humane neutrophile Elastase (HNE) handelt.

3. Deuteriumoxid zur Verwendung nach einem der vorangehenden Ansprüche, wobei Deuteriumoxid in Kombination mit mindestens einem weiteren pharmazeutischen Wirkstoff und/oder mindestens einem weiteren nicht-pharmazeutischen Wirkstoff verwendet wird.

4. Deuteriumoxid zur Verwendung nach Anspruch 3, wobei der mindestens eine weitere pharmazeutische Wirkstoff ausgewählt ist aus der Gruppe, bestehend aus Sulfonamiden, Antibiotika, insbesondere Penizillin, Kortikoiden, .Alkylfluorophosphaten, Chloromethylketonen, Sulfonylfluoriden, Trifluoromethylketonen, Borsäureestern, Aldehyden, kurzkettigen Peptiden, insbesondere mit weniger als 10 Aminosäuren, Cytostatika, Chemotherapeutika, synthetische und pflanzliche Wirkstoffe mit entzündungshemmender Wirkung.

5. Deuteriumoxid zur Verwendung nach Anspruch 3, wobei der mindestens eine weitere nicht-pharmazeutische Wirkstoff ausgewählt ist aus der Gruppe, bestehend aus pharmazeutisch verträglichen anorganischen oder organischen Säuren oder Basen, Polymeren, Copolymeren, Block-Copolymeren, Einfachzucker, Mehrfachzucker, ionische und nicht-ionische Tenside oder Lipiden, pharmakologisch unbedenkliche Salze, Geschmackstoffe, Vitamine, Tocopherole, Provitamine, Antioxidantien, Stabilisatoren und Konservierungsstoffe sowie Mischungen hiervon.

6. Deuteriumoxid zur Verwendung nach einem der vorangehenden Ansprüche, wobei Deuteriumoxid topisch, transdermal, nasal, rectal systemisch, parenteral, über eine Perfusion, über ein Endoskop oder als Aerosol oder Trockenpulverformulierung verabreicht wird.

7. Deuteriumoxid zur Verwendung nach einem der vorangehenden Ansprüche, wobei Deuteriumoxid als Formulierung verabreicht wird.

8. Deuteriumoxid zur Verwendung nach Anspruch 7, wobei die Formulierung eine Salbe, eine Creme, eine Lotion, eine Emulsion oder ein Gel oder ein Hydrogel ist.

## Claims

1. Deuterium oxide for use in the prophylaxis and/or therapy of elastase-associated pulmonary diseases, selected from the group consisting of chronic obstructive pulmonary disease (COPD), cystic fibrosis, chronic bronchitis, pulmonary fibrosis, acute respiratory syndrome, pulmonary emphysema and haemorrhage, the activity of the elastase being impeded or inhibited.

2. Deuterium for use according to claim 1, wherein the elastase is the human neutrophil elastase (HNE).

3. Deuterium oxide for use according to either of the preceding claims, wherein deuterium oxide is used in combination with at least one further pharmaceutical active ingredient and/or at least one further non-pharmaceutical active ingredient.

4. Deuterium oxide for use according to claim 3, wherein the at least one further pharmaceutical active ingredient is selected from the group consisting of sulphonamides, antibiotics, in particular penicillin, corticoids, alkyl fluorophosphates, chloromethyl ketones, sulphonyl fluorides, trifluoromethyl ketones, boric acid esters, aldehydes, short-chain peptides, in particular with less than 10 amino acids, cytostatics, chemotherapeutics, synthetic and plant active ingredients with an inflammation-inhibiting effect.

5. Deuterium oxide for use according to claim 3, wherein the at least one further non-pharmaceutical active ingredient is selected from the group consisting of pharmaceutically compatible inorganic or organic acids or bases, polymers, copolymers, block copolymers, monosaccharides , polysaccharides, ionic and nonionic surfactants or lipids, pharmacologically acceptable salts, flavourings, vitamins, tocopherols, provitamins, antioxidants, stabilisers and preservatives as well as mixtures thereof.

6. Deuterium oxide for use according to any one of the preceding claims, wherein deuterium oxide is administered topically, transdermally, nasally, rectally systemically, parenterally, by means of a perfusion, by means of an endoscope or as an aerosol or dry powder formulation.

7. Deuterium oxide for use according to any one of the preceding claims, wherein deuterium oxide is administered as a formulation.

8. Deuterium oxide for use according to claim 7, wherein the formulation is an ointment, a cream, a lotion, an emulsion or a gel or a hydrogel.

## Revendications

1. Oxyde de deutérium destiné à être utilisé pour la prophylaxie et/ou la thérapie de troubles pulmonaires liés à l'élastase, choisis parmi le groupe constitué par la maladie pulmonaire obstructive chronique (MPOC), la mucoviscidose, la bronchite chronique, la fibrose pulmonaire, le syndrome respiratoire sévère, l'emphysème pulmonaire et l'hémorragie, sachant que l'activité de l'élastase est freinée ou inhibée.

2. Oxyde de deutérium destiné à être utilisé selon la revendication 1, sachant que l'élastase est l'élastase neutrophile humaine (HNE).

3. Oxyde de deutérium destiné à être utilisé selon l'une quelconque des revendications précédentes, sachant que l'oxyde de deutérium est utilisé en combinaison avec au moins un autre principe actif pharmaceutique et/ou au moins un autre principe actif non pharmaceutique.

4. Oxyde de deutérium destiné à être utilisé selon la revendication 3, sachant que l'autre principe actif pharmaceutique au moins au nombre de un est choisi parmi le groupe constitué des sulfonamides, des antibiotiques, en particulier de la pénicilline, des corticoïdes, des fluorophosphates d'alkyle, des chlorométhylcétones, des fluorures de sulfonyle, des trifluorométhyl-cétones, des esters d'acide borique, des aldéhydes, des peptides à chaîne courte, en particulier comprenant moins de 10 acides aminés, des cytostatiques, des agents chimiothérapeutiques, des principes actifs synthétiques et végétaux ayant une action anti-inflammatoire.

5. Oxyde de deutérium destiné à être utilisé selon la revendication 3, sachant que l'autre principe actif non pharmaceutique au moins au nombre de un est choisi parmi le groupe constitué d'acides ou de bases inorganiques ou organiques pharmaceutiquement acceptables, de polymères, de copolymères, de copolymères blocs, de monosaccharides, d'oligosaccharides, d'agents tensio-actifs ou de lipides ioniques et non ioniques, de sels pharmacologiquement inoffensifs, d'agents aromatisants, de vitamines, de tocophérols, de provitamines, d'antioxydants, d'agents stabilisateurs et d'agents conservateurs ainsi que des mélanges de ces derniers.

6. Oxyde de deutérium destiné à être utilisé selon l'une quelconque des revendications précédentes, sachant que l'oxyde de deutérium est administré par voie topique, transdermale, nasale, systémique rectale, parentérale, par l'intermédiaire d'une perfusion, d'un endoscope ou sous la forme d'un aérosol ou d'une formulation de poudre sèche.

7. Oxyde de deutérium destiné à être utilisé selon l'une quelconque des revendications précédentes, sachant que l'oxyde de deutérium est administré sous la forme d'une formulation.

8. Oxyde de deutérium destiné à être utilisé selon la revendication 7, sachant que la formulation est une pommade, une crème, une lotion, une émulsion ou un gel ou un hydrogel.
